(19) Europäisches Patentamt
European Patent Office
Office européen des brevets

(11) Veröffentlichungsnummer: **0 397 602 A1**

(12) # EUROPÄISCHE PATENTANMELDUNG

(21) Anmeldenummer: **90810339.3**

(51) Int. Cl.5: **C07D 207/24, A01N 25/32**

(22) Anmeldetag: **03.05.90**

(30) Priorität: **12.05.89 CH 1797/89**

(43) Veröffentlichungstag der Anmeldung:
**14.11.90 Patentblatt 90/46**

(84) Benannte Vertragsstaaten:
**AT BE CH DE DK ES FR GB GR IT LI LU NL SE**

(71) Anmelder: **CIBA-GEIGY AG**
**Klybeckstrasse 141**
**CH-4002 Basel(CH)**

(72) Erfinder: **Lang, Robert Werner, Dr.**
**Hagenbachweg 10**
**CH-4133 Pratteln(CH)**
Erfinder: **Müller, Urs, Dr.**
**Drosselstrasse 6**
**CH-4142 Münchenstein(CH)**

(54) **Neue N-Phenylpyrrolidine.**

(57) Die 1-(3,5-Bis-trifluormethylphenyl)-2-thioxo-pyrrolidin-4-carbonsäurederivate der untenstehenden Formel I sind zum Schützen von Kulturpflanzen gegen die phytotoxische Wirkung von Herbiziden sowie zur Regulierung des Pflanzenmwachstums geeignet.

Die 1-(3,5-Bis-trifluormethylphenyl)-2-thioxo-pyrrolidin-4-carbonsäurederivate entsprechen der Formel I

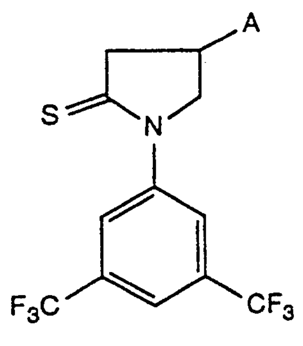

(I)

worin
A für -$COOR_1$, -$COSR_1$, -$COO^{\ominus}M^{\oplus}$, -$CONR_2R_3$ oder -COCl;
$R_1$ für Wasserstoff, $C_1$-$C_4$-Alkyl, $C_2$-$C_6$-Alkenyl oder $C_2$-$C_6$-Alkinyl;
$R_2$ und $R_3$ unabhängig voneinander für Wasserstoff, $C_1$-$C_4$-Alkyl oder $C_3$-$C_7$-Cycloalkyl;
oder $R_2$ und $R_3$ zusammen mit dem Stickstoffatom, an welches sie gebunden sind, für einen gegebenenfalls bis zu dreifach durch $C_1$-$C_4$-Alkyl substituierten gesättigten 3- bis 7-gliedrigen Heterocyclus, der noch ein weiteres Heteroatom ausgewählt aus der Gruppe O, N und S enthalten kann, stehen; und $M^{\oplus}$ das Aequivalent eines Alkali- oder Erdalkalimetallkations oder $HN^{\oplus}(R_2)_3$ bedeutet, sowie deren Isomere in optisch reiner oder angereicherter Form.

EP 0 397 602 A1

## Neue N-Phenylpyrrolidine

Die vorliegende Erfindung betrifft neue 1-(3,5-Bis-trifluormethylphenyl)-2-thioxopyrrolidin-4-carbonsäurederivate, Verfahren zu deren Herstellung, diese Wirkstoffe enthaltende Mittel, sowie die Verwendung dieser Wirkstoffe und Mittel zum Schützen von Kulturpflanzen gegen die phytotoxische Wirkung von Herbiziden und zur Regulierung des Pflanzenwachstums.

Es ist bekannt, dass Herbizide aus den Stoffklassen der Sulfonylharnstoffe und Halogenacetanilide bei der Anwendung in wirksamer Dosis gelegentlich neben den zu bekämpfenden Unkräutern auch die Kulturpflanzen in gewissem Masse schädigen. Ueberdosen werden oft ungewollt und zufälligerweise appliziert, wenn sich Randzonen beim streifenweisen Spritzen überdecken, sei es durch Windeinwirkung oder durch falsches Einschätzen der Breitenwirkung des Spritzgerätes. Es können klimatische Verhältnisse oder eine Bodenbeschaffenheit vorliegen, so dass die für normale Bedingungen empfohlene Herbizidmenge als Ueberdosis wirkt. Die Qualität des Saatgutes kann bei der Herbizidverträglichkeit auch eine Rolle spielen. Um diesem Problem zu begegnen, sind schon verschiedene Stoffe vorgeschlagen worden, welche befähigt sind, die schädigende Wirkung des Herbizids auf die Kulturpflanze spezifisch zu antagonisieren, das heisst die Kulturpflanze zu schützen, ohne dabei die Herbizidwirkung auf die zu bekämpfenden Unkräuter merklich zu beeinflussen. Dabei hat es sich gezeigt, dass die vorgeschlagenen Gegenmittel sowohl bezüglich der Kulturpflanzen als auch bezüglich des Herbizids und gegebenenfalls auch in Abhängigkeit von der Applikationsart oft sehr artspezifisch wirken, das heisst ein bestimmtes Gegenmittel eignet sich oft nur für eine bestimmte Kulturpflanze und einige wenige herbizide Stoffklassen.

So beschreibt die Britische Patentschrift 1,277,557 die Behandlung von Samen oder Sprösslingen von Weizen und Sorghum mit gewissen Oxamsäureestern und Amiden zum Schutz vor dem Angriff durch "ALACHLOR" (N-Methoxymethyl-N-chloracetyl-2,6-diäthylanilin). Im US-Patent 4,618,331 werden Benzoxazinderivate mit Schutzwirkung vor der herbiziden Wirkung von Halogenacetaniliden und Sulfonylharnstoffen offenbart. Zum Schutz gegen Sulfonylharnstoffherbizide werden als Gegenmittel in der EP-A-122 231 Benzoyloximäther und in der EP-A-147 365 Phenylglyoxylsäurenitril-oxim, Naphthalindicarbonsäureanhydrid, ein Thiazolcarbonsäureester sowie Dichloracetamide vorgeschlagen. Ferner können Maispflanzen gemäss der DE-OS 2,402,983 wirksam vor Schädigung durch Chloracetanilide geschützt werden, indem man dem Boden als Gegenmittel ein N-disubstituiertes Dichloracetamid zuführt. Derartige Verbindungen werden gemäss DE-OS 2,828,265 und 2,828,293 auch als Antidotes gegen herbizide Acetanilide verwendet.

Die US-Patentschrift Nr. 4,013,445 offenbart 1-(Bis-trifluormethylphenyl)-2-oxo-pyrrolidin-4-carbonsäurederivate als Herbizide und zur Regulierung des Pflanzenwachstums.

Aus der französischen Patentschrift 1.363.615 und der US Patentschrift 3.136.620 ist die Verwendung von 1-Phenyl-2-oxo-pyrrolidin-4-carbonsäuren sowie deren Derivate, die am Phenylkern unsubstituiert oder durch Halogenatome und/oder eine Trifluormethylgruppe substituiert sind, als Aktivsubstanzen zur Beeinflussung des Pflanzenwachstums bekannt.

Es wurde nun gefunden, dass sich überraschenderweise eine Gruppe von N-Phenylpyrrolidinen hervorragend dazu eignet, Kulturpflanzen gegen schädigende Wirkung von Sulfonylharnstoffherbiziden und Chloracetanilidherbiziden zu schützen.

Ferner weisen diese N-Phenylpyrrolidine sehr gute pflanzenwuchsregulierende Eigenschaften auf.

Die erfindungsgemäss vorgeschlagenen 1-(3,5-Bis-trifluormethylphenyl)-2-thioxo-pyrrolidin-4-carbonsäurederivate entsprechen der Formel I

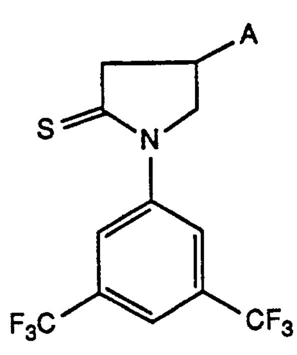

(I)

worin

A für $-COOR_1$, $-COSR_4$, $-COO^{\ominus}M^{\oplus}$, $-CONR_2R_3$ oder -COCl;

$R_1$ für Wasserstoff, $C_1$-$C_4$-Alkyl, $C_2$-$C_6$-Alkenyl oder $C_2$-$C_6$-Alkinyl;
$R_2$ und $R_3$ unabhängig voneinander für Wasserstoff, $C_1$-$C_4$-Alkyl oder $C_3$-$C_7$-Cycloalkyl;
oder $R_2$ und $R_3$ zusammen mit dem Stickstoffatom, an welches sie gebunden sind, für einen gegebenenfalls bis zu dreifach durch $C_1$-$C_4$-Alkyl substituierten gesättigten 3- bis 7-gliedrigen Heterocyclus, der noch ein weiteres Heteroatom ausgewählt aus der Gruppe O, N und S enthalten kann, stehen; und $M^{\oplus}$ das Aequivalent eines Alkali- oder Erdalkalimetallkations oder $HN^{\oplus}(R_2)_3$ bedeutet, sowie deren Isomere in optisch reiner oder angereicherter Form.

Unter Alkyl ist geradkettiges oder verzweigtes Alkyl zu verstehen, z.B. Methyl, Ethyl, n-Propyl, i-Propyl, n-Butyl, i-Butyl, sec.-Butyl oder tert. Butyl.

Unter Alkenyl ist geradkettiges oder verzweigtes Alkenyl zu verstehen; wie zum Beispiel: Vinyl, Allyl, 2-Propenyl, Methallyl, 3-Butenyl, 2-Butenyl, 3-Pentenyl, 2-Methyl-4-pentenyl, 3-Hexenyl.

In den Definitionen ist unter Alkinyl geradkettiges oder verzweigtes Alkinyl zu verstehen; z.B.: Propargyl, Aethinyl, 2-Propinyl, 3-Butinyl, 2-Methyl-3-pentinyl, 1,2-Dimethyl-3-butinyl.

Cycloalkyl steht beispielsweise für Cyclopropyl, Dimethylcyclopropyl, Cyclobutyl, Cyclopentyl, Methylcyclopentyl, Cyclohexyl oder Cycloheptyl, vorzugsweise aber für Cyclopropyl, Cyclopentyl oder Cyclohexyl.

Als Kationen $M^{\oplus}$ kommen insbesondere die von Alkalimetallen wie beispielsweise Lithium, Natrium oder Kalium und die von Erdalkalimetallen wie beispielsweise Magnesium oder Calcium in Betracht. Insbesondere steht $M^{\oplus}$ für $HN^{\oplus}(R_2)_3$, wobei die Substituenten $R_2$ gleich oder verschieden sein können, oder für die Kationen von Natrium oder Kalium.

Wenn A den Amidrest $-CONR_2R_3$ bedeutet, sind insbesondere die nachstehend genannten Reste bevorzugt: $-CONH_2$, $-CON(CH_3)_2$, $CON(C_2H_5)_2$,

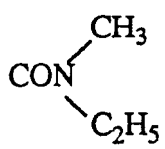 , 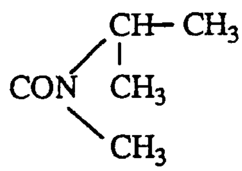 und 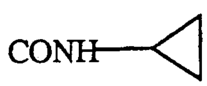 .

Beispiele für durch die Substituenten $R_2$ und $R_3$ gemeinsam mit dem sie tragenden Stickstoffatom gebildeten Heterocyclen sind Pyrrolidin, Piperazin, 2-Methylpiperazin, Piperidin und Morpholin.

Bevorzugte Verbindungen der Formel I sind jene, in denen A für $-COOR_1$ oder $-COO^{\ominus}M^{\oplus}$ steht. Aus dieser Gruppe sind als Einzelverbindungen zu nennen: 1-(3,5-Bis-trifluormethylphenyl)-2-thioxo-pyrrolidin-4-carbonsäure, 1-(3,5-Bis-trifluormethylphenyl)-2-thioxo-pyrrolidin-4-carbon säuremethylester.

Eine besonders herausragende Gruppe von Verbindungen der Formel I bilden die optisch aktiven Verbindungen der Formeln (R)-I und (S)-I

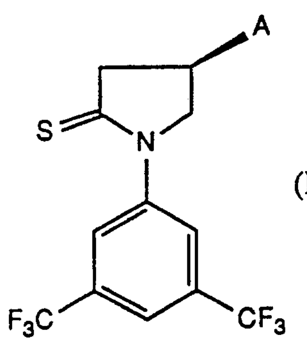
(R)-(I)

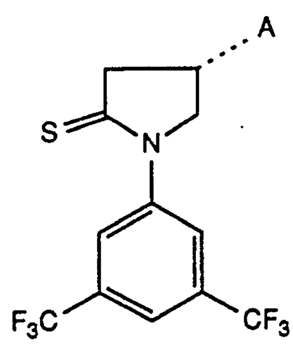
(S)-(I)

worin A die unter Formel I angegebene Bedeutung hat. Aus dieser Gruppe fallen durch ihre gute biologische Wirkung jene Verbindungen ins Auge, bei denen A für $-COOR_1$ oder $-COO^{\ominus}M^{\oplus}$ steht. Als bevorzugte Einzelverbindungen der Formeln (R)-I und (S)-I sind zu nennen:
4-(R)-1-(3,5-Bis-trifluormethylphenyl)-2-thioxo-pyrrolidin-4-carbonsäure,
4-(R)-1-(3,5-Bis-trifluormethylphenyl)-2-thioxo-pyrrolidin-4-carbonsäuremethylester,
4-(S)-1-(3,5-Bis-trifluormethylphenyl)-2-thioxo-pyrrolidin-4-carbonsäure,
4-(S)-1-(3,5-Bis-trifluormethylphenyl)-2-thioxo-pyrrolidin-4-carbonsäuremethylester.

Eine in der Wirkungsweise besonders herausragende Verbindung ist 4-(R)-1-(Bis-trifluormethylphenyl)-2-thioxo-pyrrolidin-4-carbonsäuremethylester.

Die erfindungsgemässen Verbindungen der Formel I können auf an sich bekannte Weise hergestellt werden, indem man, im Falle der racemischen Verbindungen der Formel I, ein aus USP 4,013,445 bekanntes racemisches 1-(3,5-Bis-trifluormethylphenyl)-2-oxo-pyrrolidin der Formel II

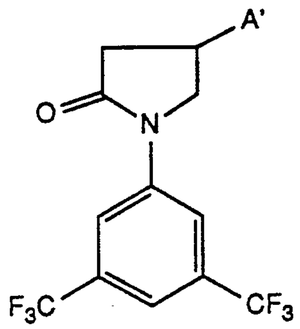

(II)

worin A′ für -COOR₁ und R₁ für C₁-C₄-Alkyl steht, mit einem zur Einführung der Thioxogruppe befähigten Reagenz zu den entsprechenden racemischen 1-(3,5-Bis-trifluormethylphenyl)-2-thioxo-pyrrolidin-4-carbonsäureestern der Formel Ia

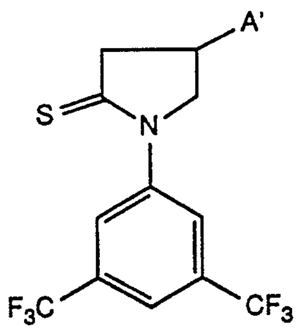

(Ia)

worin A′ für -COOR₁ und R₁ für C₁-C₄-Alkyl steht, umsetzt, und diese Verbindungen gegebenenfalls anschliessend auf an sich bekannte Weise in die anderen definitionsgemässen Derivate der Formel I, worin A die unter Formel I angegebene Bedeutung hat, überführt.

Die optisch aktiven Isomere der Formel I, dargestellt durch die Unterformeln (R)-I und (S)-I, bilden ebenfalls einen Gegenstand der vorliegenden Erfindung. Sie können auf an sich bekannte Weise hergestellt werden, indem man eine aus USP 4,013,445 bekannte racemische 1-(3,5-Bis-trifluormethylphenyl)-2-oxo-pyrrolidin-4-carbonsäure der Formel IIa

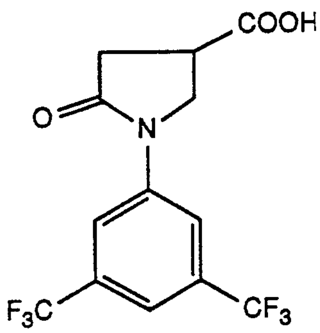

(IIa)

durch die an sich bekannte Methode der fraktionierten Kristallisation in Gegenwart eines chiralen Hilfsstoffes wie beispielsweise einer optisch aktiven Base inbesondere eines optisch aktiven 1-Phenylethylamins in die optisch aktive 4-(R)-(-)-1-(3,5-Bis-trifluormethylphenyl)-2-oxo-pyrrolidin-4-carbonsäure der Formel (R)-IIa

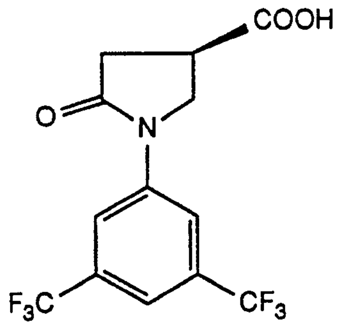

(R)-(IIa)

und die optisch aktive 4-(S)-(+)-1-(3,5-Bis-trifluormethylphenyl)-2-oxo-pyrrolidin-4-carbonsäure der Formel (S)-IIa

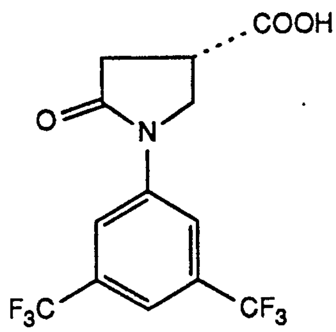

(S)-(IIa)

auftrennt, diese Isomere gegebenenfalls auf an sich bekannte Weise in die definitionsgemässen Derivate der Formel (R)-IIb und (S)-IIb

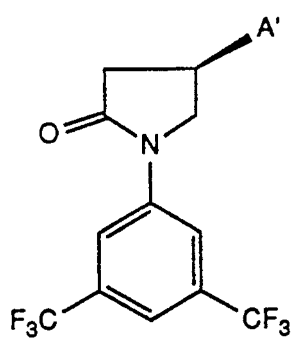

(R)-(IIb)

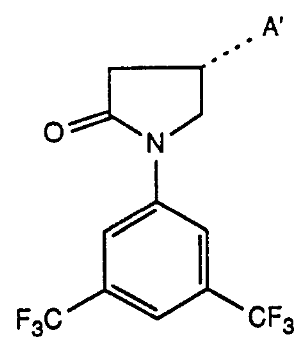

(S)-(IIb)

worin A′ für -$COOR_1$ und $R_1$ für $C_1$-$C_4$-Alkyl steht,
überführt, um anschliessend durch Umsetzung mit einem zur Einführung der Thioxogruppe befähigten Reagenz die entsprechenden Verbindungen der Formeln (R)-Ia und (S)-Ia

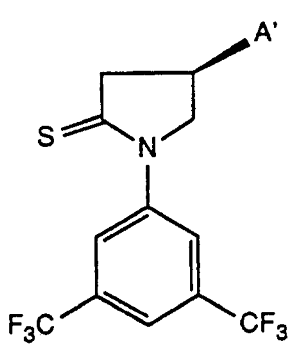

(R)-(Ia)

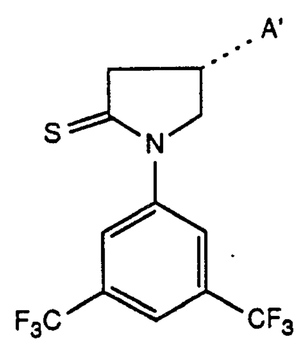

(S)-(Ia)

worin A′ für -$COOR_1$ und $R_1$ für $C_1$-$C_4$-Alkyl steht, zu erhalten, welche gegebenenfalls anschliessend auf an

sich bekannte Weise in die anderen definitionsgemässen Derivate der Formeln (R)-I und (S)-I

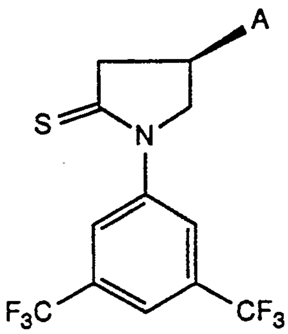

(R)-(I)

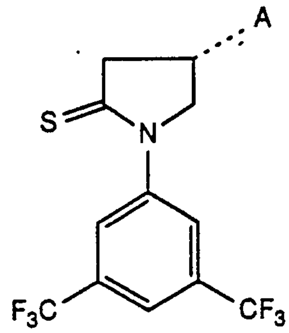

(S)-(I)

worin A die unter Formel I angegebene Bedeutung hat, überführt werden können.

Geeignete Reagenzien zur Einführung der Thioxogruppe sind beispielsweise Phosphorpentasulfid sowie die daraus hergeleiteten Arylthionophosphinsulfide. Derartige Reagenzien sind in Tetrahedron **41**, 5061-5087 (1985) beschrieben.

Als besonders gut geeignetes Reagenz zur Einführung der Thioxogruppe hat sich 2,4-Bis-(4-methoxyphenyl)-1,3-dithia-2,4-diphosphetan-2,4-disulfid (Lawesson's Reagenz) erwiesen.

Die Zwischenprodukte der Formeln (R)-IIa, (S)-IIa, (R)-IIb und (S)-IIb sind neu und bilden ebenfalls einen Gegenstand der vorliegenden Erfindung.

Die Ueberführung der 4-(R)-(-)-1-(3,5-Bis-trifluormethylphenyl)-2-oxo-pyrrolidin-4-carbonsäure der Formel (R)-IIa und der 4-(S)-(+)-1-(3,5-Bis-trifluormethylphenyl)-2-oxo-pyrrolidin-4-carbonsäure der Formel (S)-IIa in die anderen definitionsgemässen Derivate der Formeln (R)-IIb bzw. (S)-IIb erfolgt nach an sich bekannter Weise, z.B.:
durch Umsetzung der freien Carbonsäuren oder der entsprechenden Säurechloride mit Alkoholen $R_1OH$ oder durch Veresterung der freien Carbonsäure nach an sich bekannten Verfahren.

Die Ueberführung der racemischen 1-(3,5-Bis-trifluormethylphenyl)-2-thioxo-pyrrolidin-4-carbonsäureester der Formel Ia sowie deren optisch aktiven Isomeren der Formeln (R)-Ia und (S)-Ia in die anderen definitionsgemässen Derivate der Formel I erfolgt nach an sich bekannter Weise, z.B.:
zu den freien Carbonsäuren (A = -COOH) durch saure Hydrolyse der Ester der Formeln II, (R)-IIb oder (S)-IIb.
zu Säurechloriden (A = -COCl)
durch Umsetzung der freien Carbonsäuren mit geeigneten Chlorierungsmitteln, wie Thionylchlorid, Oxalylchlorid, Phosgen oder $PCl_5$.
zu Amiden

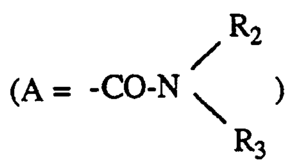

durch Umsetzung der Säureester, Säurechloride oder freien Carbonsäuren mit Aminen

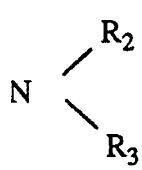

zu Thioestern (A = $-COSR_1$)
durch Umsetzung der Säurechloride mit Mercaptanen $HSR_1$;
zu Alkalimetall- oder Erdalkalimetallsalzen

durch Umsetzung der freien Carbonsäuren mit Alkalimetall- oder Erdalkalimetallhydroxiden, -alkoholaten oder -carbonaten, wie Na, K-, Li-, Ca- und Mg-hydroxid, Natrium- und Kaliummethylat und -ethylat;
zu Aminsalzen
durch Umsetzung der freien Carbonsäure mit Aminen $N(R_2)_3$.

Die Verbindungen der Formel I können dazu verwendet werden, Kulturpflanzen vor der schädigenden Wirkung von Herbiziden zu schützen. Derartige Verbindungen werden auch als "Gegenmittel", "Antidote" oder "Safener" bezeichnet.

Ein Gegenmittel oder Antidote der Formel I kann je nach Anwendungszweck zur Vorbehandlung des Saatgutes der Kulturpflanze (Beizung des Samens oder der Stecklinge) eingesetzt oder vor oder nach der Saat in den Boden gegeben werden. Es kann aber auch für sich allein oder zusammen mit dem Herbizid vor oder nach dem Auflaufen der Pflanzen appliziert werden. Die Behandlung der Pflanze oder des Saatgutes mit dem Antidote kann daher grundsätzlich unabhängig vom Zeitpunkt der Applikation des Herbizids erfolgen. Die Behandlung der Pflanze kann jedoch auch durch gleichzeitige Applikation von Herbizid und Gegenmittel (Tankmischung) erfolgen. Die preemergente Behandlung schliesst sowohl die Behandlung der Anbaufläche vor der Aussaat (ppi = pre plant incorporation) als auch die Behandlung der angesäten, aber noch nicht bewachsenen Anbauflächen ein.

Die Aufwandmengen des Gegenmittels im Verhältnis zum Herbizid richten sich weitgehend nach der Anwendungsart. Bei einer Feldbehandlung, bei der Herbizid und Gegenmittel entweder gleichzeitig (Tankmischung) oder separat appliziert werden, liegt das Verhältnis der Mengen von Gegenmittel zu Herbizid im Bereich von 1:100 bis 5:1. In der Regel wird bei einem Mengenverhältnis von Gegenmittel zu Herbizid von 5:1 bis 1:50 die volle Schutzwirkung erreicht. Bei der Samenbeizung und ähnlichen gezielten Schutzmassnahmen werden jedoch weit geringere Mengen Gegenmittel im Vergleich mit den später pro Hektar Anbaufläche verwendeten Mengen an Herbizid benötigt. Im allgemeinen werden bei der Samenbeizung pro kg Samen 0,1-10 g Gegenmittel benötigt. In der Regel wird mit 0,1-5 g Gegenmittel pro kg Samen bereits die volle Schutzwirkung erreicht. Falls das Gegenmittel kurz vor der Aussaat durch Samenquellung (seed soaking) appliziert werden soll, so werden zweckmässig Lösungen des Gegenmittels verwendet, welche den Wirkstoff in einer Konzentration von 1-10000 ppm enthalten. In der Regel wird mit Konzentrationen des Gegenmittels von 100-1000 ppm die volle Schutzwirkung erreicht.

In der Regel liegt zwischen protektiven Massnahmen, wie Samenbeizung und Behandlung von Stecklingen mit einem Gegenmittel der Formel I und der möglichen späteren Feldbehandlung mit Herbiziden ein längerer Zeitraum. Vorbehandeltes Saat- und Pflanzengut kann später in der Landwirtschaft, im Gartenbau und in der Forstwirtschaft mit unterschiedlichen Chemikalien in Berührung kommen. Die Erfindung bezieht sich daher auch auf protektive Mittel für Kulturpflanzen, die als Wirkstoff ein Gegenmittel der Formel I zusammen mit üblichen Trägerstoffen enthalten. Solche Mittel können gegebenenfalls zusätzlich jene Herbizide enthalten, vor deren Einfluss die Kulturpflanze geschützt werden soll. Ebenfalls Gegenstand der Erfindung ist mit Wirkstoffen der Formel I vorbehandeltes Vermehrungsgut von Kulturpflanzen wie Saatgut, Setzlinge oder Stecklinge. Insbesondere geeignet sind die Wirkstoffe der Formel I zur Behandlung von Saatgut von Getreide, Soja und vorzugsweise Sorghum, Mais und Reis.

Als Kulturpflanzen gelten im Rahmen vorliegender Erfindung beispielsweise sämtliche Getreidearten, wie Weizen, Roggen, Gerste und Hafer, daneben vor allem Reis, Kulturhirse, Mais und Soja. Vorzugsweise werden von den Wirkstoffen der Formel I Hirse (Sorghum), Mais und Reis gegen die Wirkung von Sulfonylharnstoffherbiziden oder Chloracetanilidherbiziden geschützt.

Besonders gute Schutzwirkung gegen Sulfonylharnstoffherbizide und gegen Chloracetanilidherbizide werden bei Anwendung der Antidotes der Formel I in Mais, Sorghum und Reis beobachtet. Hervorragende Schutzwirkung wird bei Chloracetanilidherbiziden in Reis festgestellt. Insbesondere ist dabei der günstige Effekt derjenigen Verbindungen der Formel I herauszuheben, in denen A für $-COOR_1$ oder $-COO^{\ominus}M^{\oplus}$ steht.

Zum Schutz von Reiskulturen gegen die herbizide Wirkung von N-Chloracetyl-N-(2-n-propoxyethyl)-2,6-diethylanilin ist die Verbindung 4-(R)-1-(3,5-Bis-trifluormethylphenyl)-2-thioxo-pyrrolidin-4-carbonsäuremethylester ganz besonders gut geeignet.

Sulfonylharnstoffherbizide, deren schädigende Wirkung gegenüber Kulturpflanzen mit Hilfe der N-Acylsulfamoylphenylharnstoffe der Formel I aufgehoben werden kann, sind in letzter Zeit in grosser Zahl bekannt geworden. Aus der Vielzahl der Publikationen, welche sich der Offenbarung von herbizid wirksamen Sulfonylharnstoffderivaten widmen, sollen beispielhaft das US-Patent 4 127 405 sowie die publizierten Europäischen Patentanmeldungen EP-A-7687, EP-A-30142, EP-A-44807, EP-A-44808, EP-A-51466, EP-A-70802, EP-A-84020, EP-A-87780, EP-A-102925, EP-A-108708, EP-A-120814, EP-A-136061, EP-A-184385, EP-A-206995 und EP-A-237292 genannt sein.

Typische Vertreter von herbiziden Sulfonylharnstoffderivaten sind unter der Formel III

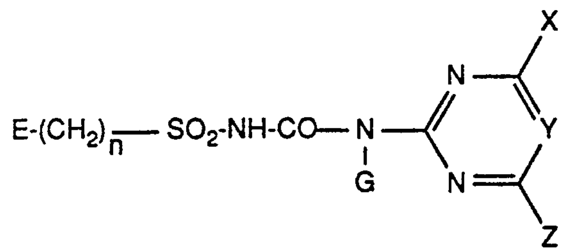

(III)

zusammengefasst, worin
E eine Gruppe

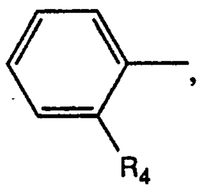 , 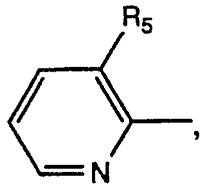 , 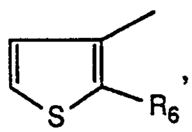 , oder 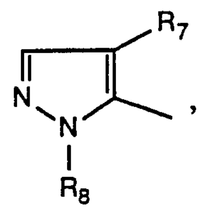 ,

n die Zahl null oder eins,
G Wasserstoff oder Methyl,
X Methoxy, Aethoxy, Difluormethoxy, Methyl oder Chlor
Y CH oder N,
Z Methoxy, Methyl, Difluormethoxy, Cyclopropyl oder Methylamino,
$R_4$ $C_2$-$C_5$-Alkoxyalkoxy, $C_1$-$C_4$-Halogenalkoxy, $C_1$-$C_4$-Halogenalkylthio, $C_2$-$C_4$-Halogenalkenyl, Chlor oder $C_1$-$C_4$-Alkoxycarbonyl,
$R_5$ Trifluormethyl oder Di($C_1$-$C_4$-alkyl)carbamoyl,
$R_6$ $C_1$-$C_4$-Alkoxycarbonyl,
$R_7$ $C_1$-$C_4$-Alkoxycarbonyl, und
$R_8$ $C_1$-$C_4$-Alkyl bedeuten.

Unter die Formel III fallen folgende herbizide Einzelwirkstoffe:
N-(3-Trifluormethylpyridin-2-ylsulfonyl)-N′-(4,6-dimethoxypy rimidin-2-yl)-harnstoff,
N-(3-Dimethylcarbamoylpyridin-2-ylsulfonyl)-N′-(4,6-dimethoxypyrimidin-2-yl)-harnstoff,
N-(1-Methyl-4-ethoxycarbonylpyrazol-2-ylsulfonyl)-N′-(4,6-dimethoxy-pyrimidin-2-yl)-harnstoff,
N-(2-Methoxycarbonylthien-3-ylsulfonyl)-N′-(4-methoxy-6-methyl-1,3,5-triazin-2-yl)-harnstoff,
N-(2-Methoxycarbonylbenzylsulfonyl)-N′-(4,6-dimethoxypyrimidin-2-yl)-harnstoff,
N-(2-Methoxycarbonylphenylsulfonyl)-N′-(4,6-bis-difluormethoxy-pyrimidin-2-yl)-harnstoff,
N-(2-Methoxycarbonylphenylsulfonyl)-N′-(4-ethoxy-6-methylamino-1,3,5-triazin-2-yl)-harnstoff,
N-(2-Methoxycarbonylphenylsulfonyl)-N′-(4-methoxy-6-methyl-1,3,5-triazin-2-yl)-harnstoff,
N-(2-Ethoxycarbonylphenylsulfonyl)-N′-(4-chlor-6-methoxypyrimidin-2-yl)-harnstoff,
N-(2-Methoxycarbonylphenylsulfonyl)-N′-(4-methoxy-6-methyl-1,3,5-triazin-2-yl)-N′-methylharnstoff,
N-(2-Methoxycarbonylphenylsulfonyl)-N′-(4,6-dimethoxypyrimidin-2-yl)-harnstoff,
N-(2-Chlorphenylsulfonyl)-N′-(4-methoxy-6-methyl-1,3,5-triazin-2-yl)-harnstoff,
N-[2-(2-Chlorethoxy)-phenylsulfonyl]-N′-(4-methoxy-6-methyl-1,3,5-triazin-2-yl)-harnstoff, und
N-[2-(2-Methoxyethoxy)-phenylsulfonyl]-N′-(4,6-dimethoxy-1,3,5-triazin-2-yl)-harnstoff.

Halogenacetanilide, deren schädigende Wirkung gegenüber Kulturpflanzen mit Hilfe der N-Phenylpyrrolidine der Formel I aufgehoben werden kann, sind ebenfalls bereits in grosser Zahl bekannt geworden. Solche Halogen- acetanilide können durch die folgende allgemeine Formel IV beschrieben werden:

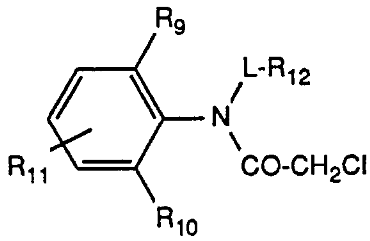

(IV)

worin L eine $C_1$-$C_4$-Alkylenbrücke,
$R_9$, $R_{10}$ und $R_{11}$ unabhängig voneinander Wasserstoff, Halogen, $C_1$-$C_4$-Alkyl, $C_1$-$C_4$-Alkoxy, $C_1$-$C_4$-Halogenalkyl, $C_2$-$C_5$-Alkoxyalkyl oder $C_2$-$C_5$-Alkylthioalkyl, und
$R_{12}$ $C_1$-$C_4$-Alkoxy, -COOH, $C_1$-$C_4$-Alkoxycarbonyl, $-CONH_2$, Wasserstoff, $C_1$-$C_4$-Alkylcarbamoyl, Di-$C_1$-$C_4$-alkylcarbamoyl, Cyan, $C_1$-$C_4$-Alkylcarbonyl,
gegebenenfalls substituiertes Benzoyl,
gegebenenfalls substituiertes Furyl,
gegebenenfalls substituiertes Thienyl,
gegebenenfalls substituiertes Pyrrolyl,
gegebenenfalls substituiertes Pyrazolyl,
gegebenenfalls substituiertes 1,3,4-Oxadiazol-2-yl,
gegebenenfalls substituiertes 1,3,4-Thiadiazol-2-yl,
gegebenenfalls substituiertes Dioxolanyl,
gegebenenfalls substituiertes Dioxanyl,
gegebenenfalls substituiertes 1,3,4-Triazol-2-yl oder
gegebenenfalls substituiertes Tetrahydrofuryl, bedeutet.

Unter die Formel IV fallen insbesondere die folgenden herbiziden Chloracetanilidderivate:
N-Aethoxymethyl-N-chloracetyl-2-ethyl-6-methylanilin,
N-Chloracetyl-N-methoxymethyl-2,6-diethylanilin,
N-Chloracetyl-N-(2-methoxyethyl)-2,6-dimethylanilin,
N-Chloracetyl-N-(2-n-propoxyethyl)-2,6-dimethylanilin,
N-Chloracetyl-N-(2-isopropoxyethyl)-2,6-dimethylanilin,
N-Chloracetyl-N-(2-methoxyethyl)-2-ethyl-6-methylanilin,
N-Chloracetyl-N-(methoxyethyl)-2,6-diethylanilin,
N-(2-Ethoxyethyl)-N-chloracetyl-2-ethyl-6-methylanilin,
N-Chloracetyl-N-(2-methoxy-1-methylethyl)-2-methylanilin,
N-Chloracetyl-N-(2-methoxy-1-methylethyl)-2,6-dimethylanilin,
N-Chloracetyl-N-(2-methoxy-1-methylethyl)-2,6-diethylanilin,
N-Chloracetyl-N-(2-methoxy-1-methylethyl)-2-ethyl-6-methylanilin,
N-(2-Ethoxyethyl)-N-chloracetyl-2,6-diethylanilin,
N-Chloracetyl-N-(2-n-propoxyethyl)-2-ethyl-6-methylanilin,
N-Chloracetyl-N-(2-n-propoxyethyl)-2,6-diethylanilin,
N-Chloracetyl-N-(2-isopropoxyethyl)-2-ethyl-6-methylanilin,
N-Ethoxycarbonylmethyl-N-chloracetyl-2,6-dimethylanilin,
N-Ethoxycarbonylmethyl-N-chloracetyl-2,6-diethylanilin,
N-Chloracetyl-N-methoxycarbonylmethyl-2,6-dimethylanilin,
N-Chloracetyl-N-(2,2-diethoxyethyl)-2,6-dimethylanilin,
N-Chloracetyl-N-(2-methoxy-1-methylethyl)-2,3-dimethylanilin,
N-(2-Ethoxyethyl)-N-chloracetyl-2-methylanilin,
N-Chloracetyl-N-(2-methoxyethyl)-2-methylanilin,
N-Chloracetyl-N-(2-methoxy-2-methylethyl)-2,6-dimethylanilin,
N-(2-Ethoxy-2-methylethyl)-N-chloracetyl-2-ethyl-6-methylanilin,
N-Chloracetyl-N-(1-ethyl-1-methoxyethyl)-2,6-dimethylanilin,
N-Chloracetyl-N-(2-methoxyethyl)-2-methoxy-6-methylanilin,
N-n-Butoxymethyl-N-chloracetyl-2-tert.-butylanilin,
N-Chloracetyl-N-(2-ethoxyethyl-2-methylethyl)-2,6-dimethylanilin,
N-Chloracetyl-N-(-2-methoxyethyl)-2-chlor-6-methylanilin,
N-(2-Ethoxyethyl)-N-chloracetyl-2-chlor-6-methylanilin,

N-(2-Ethoxyethyl)-N-chloracetyl-2,3,6-trimethylanilin,
N-Chloracetyl-1-(2-methoxyethyl)-2,3,6-trimethylanilin,
N-Chloracetyl-N-cyanomethyl-2,6-dimethylanilin,
N-Chloracetyl-N-(1,3-dioxolan-2-ylmethyl)-2,6-dimethylanilin,
N-Chloracetyl-N-(1,3-dioxolan-2-ylmethyl)-2-ethyl-6-methylanilin,
N-Chloracetyl-N-(1,3-dioxan-2-ylmethyl)-2-ethyl-6-methylanilin,
N-Chloracetyl-N-(2-furylmethyl)-2,6-dimethylanilin,
N-Chloracetyl-N-(2-furylmethyl)-2-ethyl-6-methylanilin,
N-Chloracetyl-N-(2-tetrahydrofurylmethyl)-2,6-dimethylanilin,
N-Chloracetyl-N-(N,N-dimethylcarbamoylmethyl)-2,6-dimethylanilin,
N-(n-Butoxymethyl)-N-chloracetyl-2,6-diethylanilin,
N-(2-n-Butoxyethyl)-N-chloracetyl-2,6-diethylanilin,
N-Chloracetyl-N-(2-methoxy-1,2-dimethylethyl)-2,6-dimethylanilin,
N-Chloracetyl-N-isopropyl-2,3-dimethylanilin,
N-Chloracetyl-N-isopropyl-2-chloranilin,
N-Chloracetyl-N-(1H-pyrazol-1-ylmethyl)-2,6-dimethylanilin,
N-Chloracetyl-N-(1H-pyrazol-1-ylmethyl)-2-ethyl-6-methylanilin,
N-Chloracetyl-N-(1H-1,2,4-triazol-1-ylmethyl)-2,6-dimethylanilin,
N-Chloracetyl-N-(1H-1,2,4-triazol-1-ylmethyl)-2,6-diethylanilin,
N-Benzoylmethyl-N-chloracetyl-2,6-diethylanilin,
N-Benzoylmethyl-N-chloracetyl-2-ethyl-6-methylanilin,
N-Chloracetyl-N-(5-methyl-1,3,4-oxadiazol-2-yl)-2,6-diethylanilin,
N-Chloracetyl-N-(5-methyl-1,3,4-oxadiazol-2-yl)-2-ethyl-6-methylanilin,
N-Chloracetyl-N-(5-methyl-1,3,4-oxadiazol-2-yl)-2-tert.butylanilin,
N-Chloracetyl-N-(4-chlorbenzoylmethyl)-2,6-dimethylanilin und
N-Chloracetyl-N-(1-methyl-5-methylthio-1,3,4-triazol-2-ylmethyl)-2,6-diethylanilin.

Die Antidotes der Formel I eignen sich ganz besonders, um Kulturpflanzen vor den herbiziden Wirkungen der Herbizide der Formeln III oder IV zu schützen.

Agrochemische Mittel, welche in einer gemeinsamen Formulierung neben dem Antidote der Formel I ein Sulfonylharnstoffherbizid oder ein Chloracetanilidherbizid enthalten sind zum Einsatz als selektive Herbizide in Nutzpflanzenkulturen geeignet.

Vorzugsweise enthalten die erfindungsgemässen herbiziden Mittel neben einem Antidote der Formel I einen Sulfonylharnstoff der Formel III oder ein Chloracetanilid der Formel IV.

Die angewendete Menge an Gegenmittel, sofern sie nicht auf die Samen gebeizt wird, schwankt zwischen etwa 0,01 und etwa 5 Gewichtsteilen pro Gewichtsteil Herbizid. In der Praxis ermittelt man jeweils, von Fall zu Fall, das heisst je nach verwendetem Herbizid-Typ, welches Verhältnis in Bezug auf optimale Wirkung bei der speziellen Kulturpflanze das geeignetste ist.

Die Erfindung betrifft auch ein Verfahren zur selektiven Bekämpfung von Unkräutern in Kulturpflanzenbeständen, wobei die Kulturpflanzenbestände, Teile der Kulturpflanzen oder Anbauflächen für Kulturpflanzen mit einem Herbizid und einer Verbindung der Formel I oder einem Mittel, welches diese Kombination enthält, behandelt werden. Die die Herbizid/Antidot-Kombination enthaltenden Mittel bilden ebenfalls einen Bestandteil der vorliegenden Erfindung.

Bei den zu bekämpfenden Unkräutern kann es sich sowohl um monokotyle wie um dikotyle Unkräuter handeln.

Die Erfindung betrifft auch pflanzenwachstumsregulierende Mittel, welche einen neuen Wirkstoff der Formel I enthalten, sowie Verfahren zur Hemmung des Pflanzenwuchses.

Pflanzenwachstumsregulatoren sind Substanzen, die in/an der Pflanze agronomisch erwünschte biochemische und/oder physiologische und/oder morphologische Veränderungen bewirken.

Die in den erfindungsgemässen Mitteln enthaltenen Wirkstoffe beeinflussen das Pflanzenwachstum je nach Applikationszeitpunkt, Dosierung, Applikationsart und Umweltbedingungen in verschiedener Weise. Pflanzenwuchs- regulatoren der Formel I können zum Beispiel das vegetative Wachstum von Pflanzen hemmen. Diese Art der Wirkung ist von Interesse auf Rasenflächen, im Zierpflanzenbau, in Obstplantagen, an Strassenböschungen, auf Sport- und Industrieanlagen, aber auch bei der gezielten Hemmung von Nebentrieben wie bei Tabak. Im Ackerbau führt die Hemmung des vegetativen Wachstums bei Getreide über eine Halmverstärkung zu reduziertem Lager, ähnliche agronomische Wirkung erreicht man in Raps, Sonnenblumen, Mais und anderen Kulturpflanzen. Des weiteren kann durch Hemmung des vegetativen Wachstums die Anzahl Pflanzen pro Fläche erhöht werden. Ein weiteres Einsatzgebiet von Wuchshemmern ist die selektive Kontrolle von bodendeckenden Pflanzen in Plantagen oder weitreihigen Kulturen durch

starke Wuchshemmung, ohne diese Bodendecker abzutöten, sodass die Konkurrenz gegenüber der Hauptkultur ausgeschaltet ist, die agronomisch positiven Effekte wie Erosionsverhinderung, Stickstoffbindung und Bodenlockerung aber erhalten bleiben.

Unter einem Verfahren zur Hemmung des Pflanzenwachstums ist eine Steuerung der natürlichen Pflanzenentwicklung zu verstehen, ohne den von genetischen Eigenschaften determinierten Lebenszyklus der Pflanze im Sinne einer Mutation zu verändern. Das Verfahren der Wuchsregulierung wird zu einem im Einzelfall zu bestimmenden Entwicklungszeitpunkt der Pflanze angewendet. Die Applikation der Wirkstoffe der Formel I kann vor oder nach dem Auflaufen der Pflanzen erfolgen, beispielsweise bereits auf die Samen oder die Sämlinge, auf Wurzeln, Knollen, Stengel, Blätter, Blüten oder andere Pflanzenteile. Dies kann z.B. durch Aufbringen des Wirkstoffes selbst oder in Form eines Mittels auf die Pflanzen und/oder durch Behandlung des Nährmediums der Pflanze (Erdboden) geschehen.

Bevorzugt setzt man die erfindungsgemässen Verbindungen der Formel I zur Wachstumshemmung in dikotylen Kulturen mit postemergenter Applikation ein.

Für die Verwendung der Verbindung der Formel I oder sie enthaltender Mittel zum Schützen von Kulturpflanzen gegen schädigende Wirkungen von Herbiziden sowie zur Regulierung des Pflanzenwachstums kommen verschie- dene Methoden und Techniken in Betracht, wie beispielsweise die folgenden:

i) Samenbeizung

a) Beizung der Samen mit einem als Spritzpulver formulierten Wirkstoff durch Schütteln in einem Gefäss bis zur gleichmässigen Verteilung auf der Samenoberfläche (Trockenbeizung). Man verwendet dabei etwa 0,1 bis 2,0 g Wirkstoff der Formel I (bei einer 25 %igen Formulierung: 0,4 g bis 8,0 g Spritzpulver) pro 1 kg Saatgut.

b) Beizung der Samen mit einem Emulsionskonzentrat des Wirkstoffs oder mit einer wässrigen Lösung des als Spritzpulver formulierten Wirkstoffs der Formel I nach Methode a) (Nassbeizung).

c) Beizung durch Tauchen des Saatguts in einer Brühe mit 10-1000 ppm Wirkstoff der Formel I während 1 bis 72 Stunden und gegebenenfalls nachfolgendes Trocknen der Samen (Tauchbeizung, Seed soaking).

Die Beizung des Saatguts oder die Behandlung des angekeimten Sämlings sind naturgemäss die bevorzugten Methoden der Applikation, weil die Wirkstoffbehandlung vollständig auf die Zielkultur gerichtet ist. Man verwendet in der Regel 6,0 g bis 0,01 g, vorzugsweise 2,0 bis 0,1 g Aktivsubstanz pro 1 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Applikation aus Tankmischung

Eine flüssige Aufarbeitung eines Gemisches von Gegenmittel und Herbizid (gegenseitiges Mengenverhältnis zwischen 100:1 und 1:100, vorzugsweise 10:1 und 1:10) wird verwendet, wobei die Aufwandmenge an Herbizid und Safener 0,001 bis 10 kg pro Hektar beträgt. Solche Tankmischung wird vorzugsweise vor oder nach der Aussaat appliziert oder 5 bis 10 cm tief in den noch nicht gesäten Boden eingearbeitet.

iii) Applikation in die Saatfurche

Das Gegenmittel wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

iv) Kontrollierte Wirkstoffabgabe

Der Wirkstoff wird in Lösung auf mineralische Granulatträger oder polymerisierte Granulate (Harnstoff/Formaldehyd) aufgezogen und trocknen gelassen. Gegebenenfalls kann ein Ueberzug aufgebracht werden (Umhüllungsgranulate), der es erlaubt, den Wirkstoff über einen bestimmten Zeitraum dosiert abzugeben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise herge- stellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfonate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykolethergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyethylenglykolether, Polypropylen-polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1981;

H. Stache, "Tensid-Taschenbuch", 2. Aufl., C. Hanser Verlag, München, Wien, 1981;

M. and J. Ash. "Encyclopedia of Surfactants", Vol. I-III, Chemical Publishing Cc., New York, 1980-1981.

Die agrochemischen Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

Emulgierbare Konzentrate:

Aktiver Wirkstoff: 1 bis 20 %, bevorzugt 5 bis 10 %
oberflächenaktive Mittel: 5 bis 30 %, vorzugsweise 10 bis 20 %
flüssiges Trägermittel: 50 bis 94 %, vorzugsweise 70 bis 85 %

Stäubemittel:

Aktiver Wirkstoff: 0,1 bis 10 %, vorzugsweise 0,1 bis 1 %
festes Trägermittel: 99,9 bis 90 %, vorzugsweise 99,9 bis 99 %

Suspensions-Konzentrate:

Aktiver Wirkstoff: 5 bis 75 %, vorzugsweise 10 bis 50 %
Wasser: 94 bis 24 %, vorzugsweise 88 bis 30 %
oberflächenaktives Mittel: 1 bis 40 %, vorzugsweise 2 bis 30%

Benetzbares Pulver:

Aktiver Wirkstoff: 0,5 bis 90 %, vorzugsweise 1 bis 80 %
oberflächenaktives Mittel: 0,5 bis 20 %, vorzugsweise 1 bis 15 %
festes Trägermittel: 5 bis 99 %, vorzugsweise 15 bis 90 %

Granulate:

Aktiver Wirkstoff: 0,5 bis 30 %, vorzugsweise 3 bis 15 %
festes Trägermittel: 99,5 bis 70 %, vorzugsweise 97 bis 85 %.

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele:

Beispiel F 1: Formulierungsbeispiele für Wirkstoffe der Formel I (% = Gewichtsprozent)

| a) Spritzpulver | a) | b) | c) |
|---|---|---|---|
| Wirkstoff Nr. 1.01 | 20 % | 50 % | 0,5 % |
| Na-Ligninsulfonat | 5 % | 5 % | 5 % |
| Na-Laurylsulfat | 3 % | 5 % | 5 % |
| Na-Diisobutylnaphthalinsulfonat | - | 6 % | 6 % |
| Octylphenolpolyethylenglykoläther(7-8 Mol AeO) | - | 2 % | 2 % |
| Hochdisperse Kieselsäure | 5 % | 27 % | 27 % |
| Kaolin | 67 % | 10 % | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff Nr. 1.02 | 10 % | 1 % |
| Octylphenolpolyethylenglykolether (4-5 Mol AeO) | 3 % | 3 % |
| Ca-Dodecylbenzolsulfonat | 3 % | 3 % |
| Ricinusölpolyglykolether (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff Nr. 1.02 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff Nr. 1.01 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff Nr. 1.01 | 3 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| f) Suspensions Konzentrat | a) | b) |
|---|---|---|
| Wirkstoff Nr. 1.02 | 40 % | 5 % |
| Ethylenglykol | 10 % | 10 % |
| Nonylphenolpolyethylenglykolether (15 Mol AeO) | 6 % | 1 % |
| Na-Ligninsulfonat | 10 % | 5 % |
| Carboxymethylcellulose | 1 % | 1 % |
| 37 %ige wässrige Formaldehyd-Lösung Silikonöl in Form einer 75 %igen | 0,2 % | 0,2 % |
| wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) Salzlösung | |
|---|---|
| Wirkstoff Nr. 1.02 | 5 % |
| Isopropylamin | 1 % |
| Octylphenolpolyethylenglykolether (78 Mol AeO) | 3 % |
| Wasser | 91 % |

Herstellungsbeispiele:

Die folgenden Beispiele veranschaulichen die Herstellung einiger erfindungsgemässer Verbindungen.

Beispiel H1: Herstellung von 4-(R)-(-)-1-(3,5-Bis-trifluormethylphenyl)-2-oxo-pyrrolidin-4-carbonsäure:

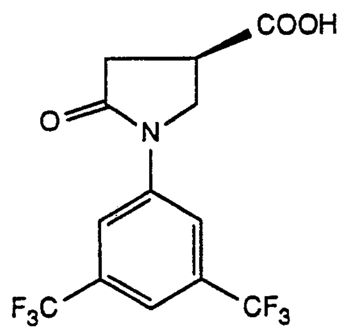

(R)-(IIa)

a) Zu einer Lösung aus 932,3g (2,734 Mol) racemischer 1-(3,5-Bis-trifluormethylphenyl)-2-oxo-pyrrolidin-4-carbonsäure in 6000 ml Ethanol gibt man bei einer Temperatur von +60 °C eine Lösung aus 331,4 g (2,734 Mol) (R)-(+)-1-Phenylethylamin in 500 ml Ethanol unter Rühren tropfenweise hinzu. Nach 10-stündigem Rühren der inzwischen auf Raumtemperatur abgekühlten Mischung wird der entstandene Niederschlag abfiltriert und mit 300 ml kaltem Ethanol gewaschen.

b) Der gemäss Beispiel H1a) erhaltene Niederschlag wird getrocknet. Nach Umkristallisieren aus 2500 ml Ethanol erhält man 369,5 g eines kristallinen Addukts mit einem Schmelzpunkt von +189 bis +190 °C.

c) 355 g der gemäss Beispiel H1b) erhaltenen Kristallfraktion werden in 1000 ml Wasser suspendiert. Anschliessend wird die Suspension mit konzentrierter Salzsäure auf einen pH-Wert von 1 bis 2 eingestellt, wobei ein Niederschlag entsteht Das Reaktionsgemisch wird mit 1500 ml Essig- säureethylester extrahiert. Anschliessend wird die organische Phase mit 2N-Salzsäure sowie mit konzentrierter Natriumchloridlösung gewaschen. Nach Trocknen über Natriumsulfat und Eindampfen der Lösung erhält man 246 g 4-(R)-(-)-1-(3,5-Bis-trifluormethylphenyl)-2-oxo-pyrrolidin-4-carbonsäure der Formel (R)-(IIa) mit einem Schmelzpunkt von +127 bis 128 °C.

Beispiel H2: Herstellung von 4-(S)-(+)-1-(3,5-Bis-trifluormethylphenyl)-2-oxo-pyrrolidin-4-carbonsäure:

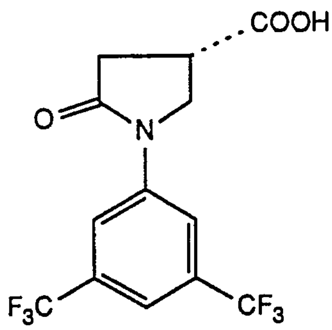

(S)-(IIa)

Das gemäss Beispiel 1a) erhaltene Filtrat wird eingedampft und anschliessend in wässrigem 2-N-Natriumhydroxid gelöst. Nach Extraktion der Lösung mit Dichlormethan wird die wässrige Phase mit konzentrierter Salzsäure auf den pH-Wert 1 bis 2 eingestellt, wobei ein Niederschlag entsteht. Nach Trocknen des Niederschlages erhält man 566 g 4-(S)-(+)-1-(3,5-Bis-trifluormethylphenyl)-2-oxo-pyrrolidin-4-carbonsäure der Formel (S)-(IIa) mit einem Schmelzpunkt von +127 bis 130 °C.

Beispiel H3: Herstellung von 4-(R)-(-)-1-(3,5-Bis-trifluormethylphenyl)-2-oxo-pyrrolidin-4-carbonsäuremethylester:

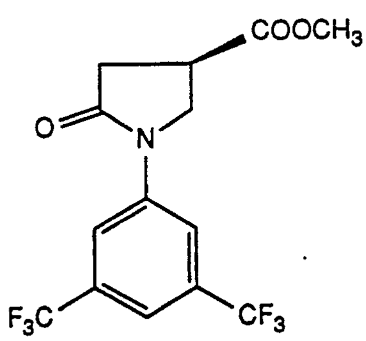

(R)-(IIb)

Eine Lösung aus 246 g gemäss Beispiel 1 hergestellter 4-(R)-(-)-1-(3,5-Bis-trifluormethylphenyl)-2-oxo-pyrrolidin-4-carbonsäure in 1600 ml Methanol wird mit 20 g stark saurem Amberlyst|[reg 15 versetzt und 20 Stunden unter Rückfluss erhitzt. Anschliessend wird die Reaktionsmischung filtriert und eingedampft. Der Rückstand wird in 2000 ml Essigsäureethylester gelöst und mit einer 10 %igen wässrigen Natriumhydrogencarbonatlösung sowie mit einer gesättigten Natriumchloridlösung gewaschen. Nach Trocknen über Natriumsulfat und Aktivkohle wird das Reaktionsgemisch filtriert. Nach Eindampfen des Filtrates auf 10 %

des ursprünglichen Volumens wird das Filtrat mit n-Hexan versetzt, wobei die Lösung in den zweiphasigen Zustand übergeht und die Kristallisation des Produkts einsetzt.

Nach Filtration erhält man 200,1 g (41,2 % d.Th.) 4-(R)-(-)-1-(3,5-Bis-trifluormethylphenyl)-2-oxo-pyrrolidin-4-carbonsäuremethylester der Formel (R)-(IIb) mit einem Drehwert von $\alpha_D^{20}$(-) 23,0° und einem Schmelzpunkt von +54 bis +55° C.

<u>Beispiel H4: Herstellung von 4-(S)-(+)-1-(3,5-Bis-trifluormethylphenyl)-2-oxo-pyrrolidin-4-carbonsäuremethylester</u>

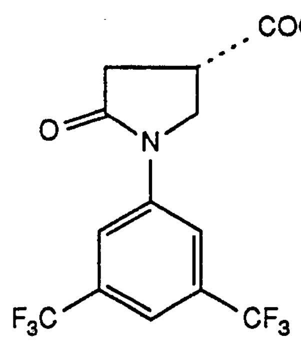

(S)-(IIb)

Die gemäss Beispiel H2 hergestellte 4-(S)-(+)-1-(3,5-Bis-trifluormethylphenyl)-2-oxo-pyrrolidin-4-carbonsäure der Formel (S)-(IIa) wird gemäss Beispiel H3 in den 4-(S)-(+)-1-(3,5-Bis-trifluormethylphenyl)-2-oxo-pyrrolidin-4-carbonsäuremethylester der Formel (S)-(IIb), der durch einen Schmelzpunkt von +54 bis +55° C und einem Drehwert von $\alpha_D^{20}$ (+) 23,0° charakterisiert ist, überführt.

<u>Beispiel H5: Herstellung von 4-(R)-(-)-1-(3,5-Bis-trifluormethylphenyl)-2-thioxo-pyrrolidin-4-carbonsäuremethylester (Verbindung Nr. 1.01):</u>

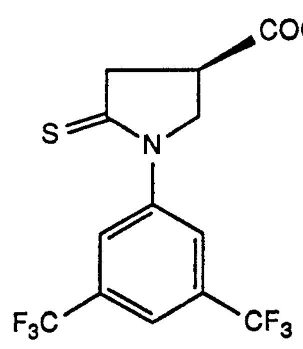

(R)-(I)

Eine Lösung aus 10,66 g (0,03 Mol) gemäss Beispiel 3 hergestelltem 4-(R)-(-)-1-(3,5-Bis-trifluormethylphenyl)-2-oxo-pyrrolidin-4-carbonsäuremethylester und 6,06 g (0,015 Mol) Lawesson's-Reagenz (2,4-Bis-(4-methoxyphenyl)-1,3-dithia-2,4-diphosphetan-2,4-disulfid) in 70 ml Toluol wird bei +80° C 90 Minuten lang erhitzt.

Anschliessend wird das Reaktionsgemisch bis zur Bildung einer dickflüssigen gelben Substanz eingeengt. Nach chromatographischer Reinigung über 780 g Siliziumdioxid in n-Hexan/Essigsäureethylester (3:1) und Umkristallisation aus einem Diethylether/n-Pentan-Gemisch erhält man nach Trocknen im Vakuum bei 40° C 10,18 g (91 % d.Th.) 4-(R)-(-)-1-(3,5-Bis-trifluormethylphenyl)-2-thioxo-pyrrolidin-4-carbonsäuremethylester (Verbindung Nr. 1.01) in Form weisser Kristalle mit einem Schmelzpunkt von +83 bis +84° C und einem Drehwert von $\alpha_D^{20}$ (-) 13,0°.

Beispiel H6: Herstellung von 4-(S)-(+)-1-(3,5-Bis-trifluormethylphenyl)-2-thioxo-pyrrolidin-4-carbonsäuremethylester (Verbindung Nr. 1.03):

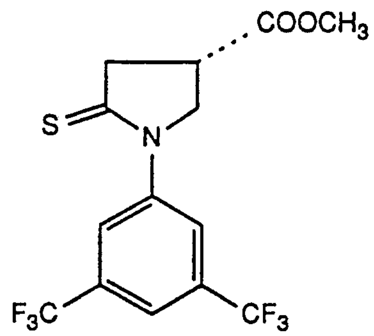

(S)-(I)

Der gemäss Beispiel H4 hergestellte 4-(S)-(+)-1-(3,5-Bis-trifluormethylphenyl)-2-oxo-pyrrolidin-4-carbonsäuremethylester der Formel (S)-(IIb) wird gemäss Beispiel H5 in den 4-(S)-(+)-1-(3,5-Bis-trifluormethylphenyl)-2-thioxopyrrolidin-4-carbonsäuremethylester der Formel (S)-(I), welcher durch einen Schmelzpunkt von + 85 bis + 87 °C und einem Drehwert von $\alpha_D^{20}$ (+) 13,0 ° charakterisiert ist, überführt.

Beispiel H7: Herstellung von racemischem 1-(3,5-Bis-trifluormethylphenyl)-2-thioxo-pyrrolidin-4-carbonsäuremet hylester (Verbindung Nr. 1.02):

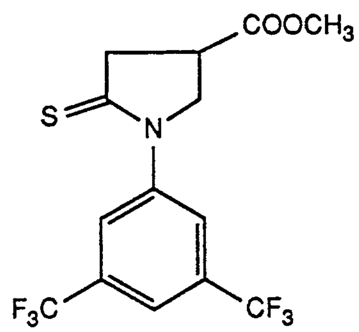

(I)

Eine Lösung aus 10.66 g (0,03 Mol) eines aus racemischer 1-(3,5-Bis-trifluormethylphenyl)-2-oxo-pyrrolidin-4-carbonsäure gemäss Beispiel 3 hergestelltem 1-(3,5-Bis-trifluormethylphenyl-2-oxo-pyrrolidin-4-carbonsäuremethylesters wird mit 6,06 g Lawesson's-Reagenz (0,015 Mol) in 70 ml Toluol 1 bis 2 Stunden auf + 80 °C erhitzt und anschliessend eingedampft. Der kristalline Rückstand wird mit n-Pentan und kleinen Mengen Diethyl- ether verrührt und bei 0 °C auf einen Saugfilter gegeben. Nach Waschen des Filterrückstandes mit einer kleinen Menge n-Pentan und Trocknen bei +40 °C im Vakuum erhält man 9,19 g (82,5 % d.Th.) des racemischen 1-(3,5-Bis-trifluormethylphenyl)-2-thioxo-pyrrolidin-4-carbonsäuremethylesters (Verbindung Nr. 1.02) in Form weisser Kristalle mit einem Schmelzpunkt von +85 bis +87 °C.

Beispiel H8: Herstellung von racemischer 1-(3,5-Bis-trifluormethylphenyl)-2-thioxo-pyrrolidin-4-carbonsäure (Verbindung Nr. 1.06)

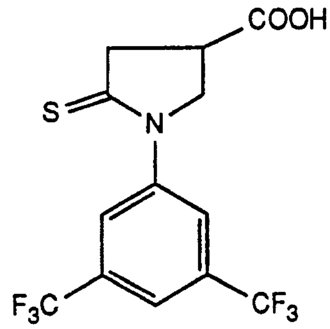

(I)

a) Herstellung von 1-(3,5-Bis-trifluormethylphenyl)-2-oxo-pyrrolidin-4-carbonsäure-tert.-butylester:

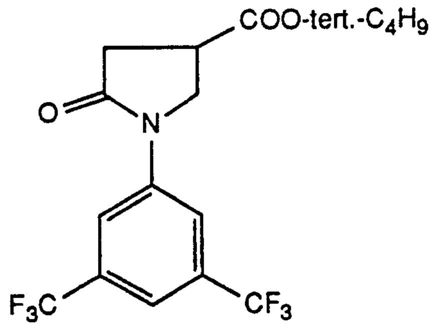

Eine Lösung von 3,41 g (0,01 Mol) racemischer 1-(3,5-Bis-trifluormethylphenyl)-2-oxo-pyrrolidin-4-carbonsäure in 50 ml absolutem Tetrahydrofuran wird auf 0° C gekühlt und mit 2,12 ml (0,015 Mol) 1-Chlor-1-dimethylamino-2-methyl-1-propen versetzt und 3 Stunden bei 10 - 15° C gerührt.

Nach Kühlen auf 0° C wird dem Reaktionsgemisch eine Mischung aus 2 ml (0,022 Mol) tert.-Butanol, 1,6 ml (0,02 Mol) Pyridin und 0,24 g (0,002 Mol) 4-Dimethylaminopyridin zugegeben.

Nach 12-stündigem Rühren wird mit Wasser und Dichlorethan extrahiert, die organische Phase getrocknet, gewaschen und anschliessend eingedampft.

Nach chromatographischer Reinigung mit Kieselgel/Dichlormethan erhält man 3,76 g (95 % d. Th.) 1-(3,5-Bis-trifluormethylphenyl)-2-oxo-pyrrolidin-carbonsäure-4-tert.-butylester in Form eines gelben Oeles.

b) Herstellung von 1-(3,5-Bis-trifluormethylphenyl)-2-thioxo-pyrrolidin-4-carbonsäure-tert.-butylester (Verbindung Nr. 1.18):

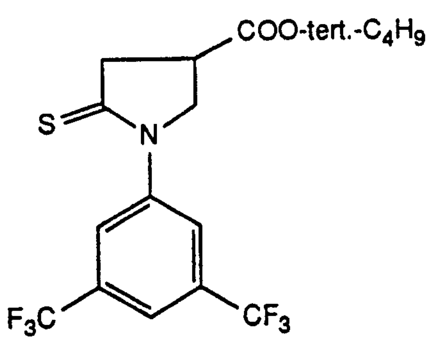

Eine Mischung aus 3 g (7,55 mMol) gemäss Beispiel H8a) hergestelltem 1-(3,5-Bis-trifluormethylphenyl)-2-oxo-pyrrolidin-4-carbonsäure-tert.-butylester und 1,53 g (3,77 mMol) Lawesson-Reagenz wird in 20 ml Toluol unter Rühren auf 80° C erhitzt. Nach 2 Stunden wird die Lösung eingedampft und der erhaltene Rückstand über Kieselgel/(Hexan: Essigsäureethylester 7:1) chromatographisch gereinigt. Aus der Lösung

kristallisiert 2,82 g (91 % d.Th.) 1-(3,5-Bis-trifluormethylphenyl)-2-thioxo-pyrrolidin-4-carbonsäure-tert.butylester (Verbindung Nr. 1.18) aus.

c) Herstellung von 1-(3,5-Bis-trifluormethylphenyl)-2-thioxo-pyrrolidin-4-carbonsäure (Verbindung Nr. 1.06):

1 g (2,42 mMol) eines gemäss Beispiel H8b) hergestellten 1-(3,5-Bis-trifluormethyl-phenyl)-2-thioxo-pyrrolidin-4-carbonsäure-tert.-butylesters wird in Trifluoressigsäure gelöst und unter vermindertem Druck bei Raumtemperatur 2 Stunden gerührt. Anschliessend wird die überschüssige Trifluoressigsäure abgedampft. Nach Umkristallisation des so erhaltenen Rückstandes aus Diethylether/n-Pentan erhält man 0,76 g (89 % d.Th.) 1-(3,5-Bis-trifluormethylphenyl)-2-thioxo-pyrrolidin-4-carbonsäure (Verbindung Nr. 1.06) in Form farbloser Kristalle mit einem Schmelzpunkt von + 141 bis + 142 °C.

Nachfolgende Beispiele für Verbindungen der Formeln I, (R)-IIb und (S)-IIb stehen stellvertretend für den beschriebenen Umfang:

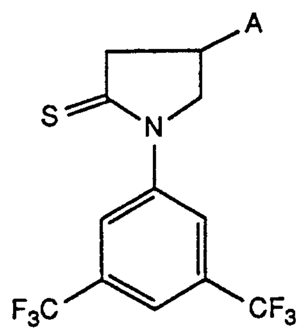

(I)

Tabelle 1:

| Verbindung Nr. | A | Schmelzpunkt [°C], Drehwert $[\alpha^{20}_{D}]$ | |
|---|---|---|---|
| 1.01 | (R)-$COOCH_3$ | + 83 bis + 84 | - 13° |
| 1.02 | ± -$COOCH_3$ | + 85 bis + 87 | - |
| 1.03 | (S)-$COOCH_3$ | + 85 bis + 87 | + 13° |
| 1.04 | (R)-COOH | + 141 bis + 142 | - 20° |
| 1.05 | (S)-COOH | + 140 bis + 142 | + 20° |
| 1.06 | ± -COOH | + 141 bis + 142 | - |
| 1.07 | (R)-$COOCH_2CH_3$ | | |
| 1.08 | (S)-$COOCH_2CH_3$ | | |
| 1.09 | ± -$COOCH_2CH_3$ | | |
| 1.10 | (R)-COO-CH-$CH_3$<br>\|<br>$CH_3$ | | |
| 1.11 | (S)-COO-CH-$CH_3$<br>\|<br>$CH_3$ | | |
| 1.12 | ± -COO-CH-$CH_3$<br>\|<br>$CH_3$ | | |
| 1.13 | (R)-COO-$CH_2CH_2CH_2CH_3$ | | |
| 1.14 | (S)-COO-$CH_2CH_2CH_2CH_3$ | | |
| 1.15 | ± -COO-$CH_2CH_2CH_2CH_3$ | | |
| 1.16 | (R)-COO-tert.-$C_4H_9$ | + 60 bis + 61 | |
| 1.17 | (S)-COO-tert.-$C_4H_9$ | + 60 bis + 62 | |
| 1.18 | ± -COO-tert.-$C_4H_9$ | + 96 bis + 97 | |
| 1.19 | (R)-$COOCH_2$-CH=$CH_2$ | + 40 bis + 42 | - 10,3° |
| 1.20 | (S)-$COOCH_2$-CH=$CH_2$ | | |

Tabelle 1:

| Verbindung Nr. | A | Schmelzpunkt [°C], Drehwert $[\alpha^{20}_{D}]$ | |
|---|---|---|---|
| 1.21 | ± -$COOCH_2$-CH=$CH_2$ | + 42 bis + 44 | |
| 1.22 | (R)-$COOCH_2$-C≡CH | + 84 bis + 86 | - 10,7° |
| 1.23 | (S)-$COOCH_2$-C≡CH | | |
| 1.24 | ± -$COOCH_2$-C≡CH | + 47 bis + 49 | |
| 1.25 | (R)-$COOCH_2$-CH=CH<br>|<br>$CH_3$ | | |
| 1.26 | (S)-$COOCH_2$-CH=CH<br>|<br>$CH_3$ | | |
| 1.27 | ± -$COOCH_2$-CH=CH<br>|<br>$CH_3$ | | |
| 1.28 | (R)-$COOCH_2$-C≡C-$CH_3$ | | |
| 1.29 | (S)-$COOCH_2$-C≡C-$CH_3$ | | |
| 1.30 | ±-$COOCH_2$-C≡C-$CH_3$ | | |
| 1.31 | (R)-COCl | | |
| 1.32 | (S)-COCl | | |
| 1.33 | ± -COCl | | |
| 1.34 | (R)-$COSCH_3$ | | |
| 1.35 | (S)-$COSCH_3$ | | |
| 1.36 | ± -$COSCH_3$ | + 108 bis + 110 | |
| 1.37 | (R)-COS-n-$C_4H_9$ | Oel | - 10,3° |
| 1.38 | (S)-COS-n-$C_4H_9$ | | |
| 1.39 | ± -COS-n-$C_4H_9$ | + 62 bis + 63 | |
| 1.40 | $CH_3$<br>/<br>(R)-CON<br>\\<br>$CH_3$ | | |

Tabelle 1:

| Verbindung Nr. | A | Schmelzpunkt [°C], Drehwert $[\alpha^{20}_{D}]$ |
|---|---|---|
| 1.41 | (S)-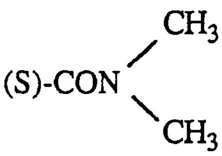 | |
| 1.42 | ± -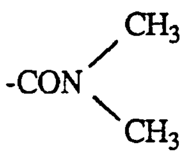 | + 120 bis + 122 |
| 1.43 | (R) $-COO^{\ominus}Na^{\oplus}$ | |
| 1.44 | (S) $-COO^{\ominus}Na^{\oplus}$ | |
| 1.45 | ± $-COO^{\ominus}Na^{\oplus}$ | + 248 bis + 250 |
| 1.46 | $[(R)-COO^{\ominus}]^{2\ominus}Ca^{2\ominus}$ | |
| 1.47 | $[(S)-COO^{\ominus}]^{2\ominus}Ca^{2\ominus}$ | |
| 1.48 | $[\pm -COO^{\ominus}]^{2\ominus}Ca^{2\ominus}$ | |
| 1.49 | (R) $-COO^{\ominus\oplus}NH(C_4H_9)_3$ | |
| 1.50 | (S) $-COO^{\ominus\oplus}NH(C_4H_9)_3$ | |
| 1.51 | ± $-COO^{\ominus\oplus}NH(C_4H_9)_3$ | Oel |
| 1.52 | (R) $-COO^{\ominus\oplus}NH_4$ | |
| 1.53 | (S) $-COO^{\ominus\oplus}NH_4$ | |
| 1.54 | ± $-COO^{\ominus\oplus}NH_4$ | |
| 1.55 | (R) 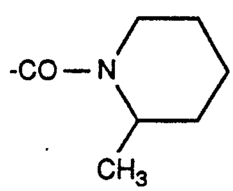 | |
| 1.56 | (S) 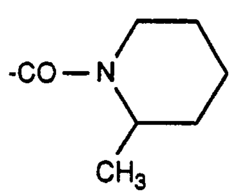 | |

Tabelle 1:

| Verbindung Nr. | A | Schmelzpunkt [°C], Drehwert $[\alpha^{20}_{D}]$ |
|---|---|---|
| 1.57 | ± 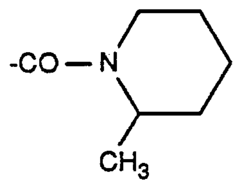 | |
| 1.58 | (R) 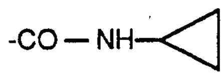 | |
| 1.59 | (S) 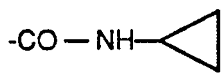 | |
| 1.60 | ± 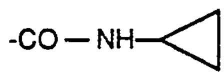 | + 172 bis + 174 |
| 1.61 | ± 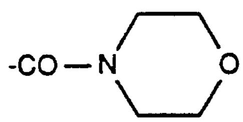 | + 120 bis + 122 |

Tabelle 2

Zwischenprodukte der Formel (R)-(IIb)

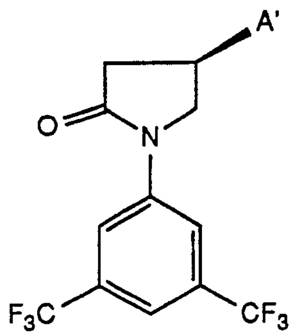

| Verbindung Nr. | A' | Schmelzpunkt [°C], Drehwert $[\alpha^{20}_{D}]$ | |
|---|---|---|---|
| 2.01 | $-COOCH_3$ | + 54 bis + 55 | - 23° |
| 2.02 | $-COOCH_2CH_3$ | | |
| 2.03 | $-COO-CH(CH_3)-CH_3$ | | |
| 2.04 | $-COO-CH_2CH_2CH_2CH_3$ | | |
| 2.05 | -COO-tert.-$C_4H_9$ | Oel | |
| 2.06 | $-COO-CH(CH_3)-CH_2-CH_3$ | | |

Tabelle 3

Zwischenprodukte der Formel (S)-(IIb)

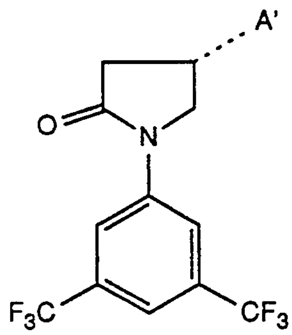

| Verbindung Nr. | A' | Schmelzpunkt [°C], | Drehwert $[\alpha^{20}_{D}]$ |
|---|---|---|---|
| 3.01 | $-COOCH_3$ | + 54 bis + 55 | + 23° |
| 3.02 | $-COOCH_2CH_3$ | | |
| 3.03 | $-COO-CH(CH_3)-CH_3$ | | |
| 3.04 | $-COO-CH_2CH_2CH_2CH_3$ | | |
| 3.05 | -COO-tert.-$C_4H_9$ | Oel | |
| 3.06 | $-COO-CH(CH_3)-CH_2-CH_3$ | | |

Biologische Beispiele:

Die Wirksamkeit der erfindungsgemässen Verbindungen der Formel I wurde wie folgt geprüft:

Beispiel B1: Safeningwirkung in Mais (Tankmix)

Zur Ueberprüfung der Safeningwirkung werden Samen von Mais (Sorte Blizzard) in Töpfen 9 x 9 cm in Erde angezogen. Die Kultur der Pflanzen erfolgt im Gewächshaus unter angepassten Temperatur- und Lichtverhältnissen. Das Giessen resp. Düngen der Pflanzen erfolgt nach Bedarf.

Die Applikation der Safenersubstanz (400 g AS/ha) erfolgt als Tankmix zusammen mit dem Herbizid mit einer Wasseraufwandmenge von 550 lt./ha im Nachauflauf (post).

Zur Erfassung der Safeningwirkung (Schutzwirkung) wird 14 Tage nach der Applikation die allgemeine Schädigung (Phytotox) an den Pflanzen bonitiert (0 % Phyto = kein Schaden, wie unbehandelte Kontrolle; 100 % Phyto = Totalschaden). Aus der Differenz der Phyto der Herbizidbehandlung allein und der

Kombination von Herbizid + Safener ergibt sich die in der Tabelle 2 aufgeführte Schutzwirkung in %.

Die Ergebnisse für das folgende Herbizid der Formel IIIa

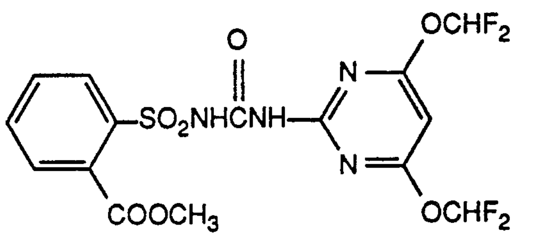

(IIIa)

N-(2-Methoxycarbonylphenylsulfonyl)-N′-(4,6-bis-difluormethoxyprimidin-2-yl)-harnstoff, und den Safenern 1-(3,5-Bis-trifluormethylphenyl)-2-thioxo-pyrrolidin-4-carbonsäuremethylester (Verbindung Nr. 1.02) und 4-(R)-1-(3,5-Bis-trifluormethylphenyl)-2-thioxo-pyrrolidin-4-carbonsäuremethylester (Verbindung Nr. 1.01) sind in Tabelle 2 zusammengestellt.

| Herbizid der Formel IIIa Aufwandmenge 75 g/ha | Schutzwirkung in % |
|---|---|
| Safener Nr. 1.01 | 12 % |
| Safener Nr. 1.02 | 12 % |

Beispiel B2: Safeningwirkung in Reis (Tankmix)

Zur Ueberprüfung der Safeningwirkung werden Samen von Reis (Sorte S-201) in Töpfen 9 x 9 cm in sumpfiger Erde angezogen. Die Kultur der Pflanzen erfolgt im Gewächshaus unter angepassten Temperatur- und Lichtverhältnissen. Das Giessen resp. Düngen der Pflanzen erfolgt nach Bedarf.

Die Applikation der Safenersubstanz (250 g AS/ha) erfolgt als Tankmix zusammen mit dem Herbizid mit einer Wasseraufwandmenge von 550 lt./ha im Vorauflauf (pre). Die Reissamen werden vor der Saat während ca. 40 Stunden vorgequollen.

Zur Erfassung der Safeningwirkung (Schutzwirkung) wird 14 Tage nach der Applikation die allgemeine Schädigung (Phytotox) an den Pflanzen bonitiert (0 % Phyto = kein Schaden, wie unbehandelte Kontrolle; 100 % Phyto = Totalschaden). Aus der Differenz der Phyto der Herbizidbehandlung allein und der Kombination von Herbizid + Safener ergibt sich die Schutzwirkung in %.

Die Ergebnisse für das Herbizid N-Chloracetyl-N-(2-n-propoxyethyl)-2,6-diethylanilin der Formel IVa

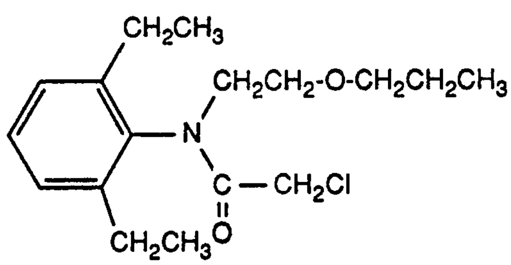

(IVa)

und den Safenern Nr. 1.01 und Nr. 1.02 sind in Tabelle 3 aufgeführt.

Tabelle 3:

| Herbizid der Formel IVa Aufwandmenge 1,5 kg/ha | Schutzwirkung in % |
|---|---|
| Safener Nr. 1.01 | 38 % |
| Safener Nr. 1.02 | 25 % |

Beispiel B3: Wuchshemmung bei tropischen Bodendecker-Leguminosen (Cover-Crops)

Die Pflanzen (z.B. Centrosema pubescens oder Psophocarpus palustris) werden in 4 cm -Torftöpfen mit Landerde (45 %), Torf (45 %) und Zonolit (10 %) durch Stecklinge vermehrt. Die Anzucht erfolgt im Gewächshaus bei einer Tagestemperatur von 27° C und einer Nachttemperatur von 23° C. Die Beleuchtungsdauer ist mindestens 14 Stunden/Tag bei einer Intensität von mindestens 7000 Lux.

Ca. 50 Tage nach Ansatz der Stecklinge erfolgt das Vertopfen in 13 cm-Töpfe, 4-5 Pflanzen/Topf. Nach weiteren 60 Tagen werden die Pflanzen auf ca. 15 cm Höhe zurückgeschnitten und appliziert. Dabei werden sie mit 0.3 bis 3 kg Wirkstoff/ha (in der Regel 25 %ig formuliert) in wässriger Spritzbrühe besprüht. Die Wasseraufwandmenge beträgt ca. 200 l/ha.

4 Wochen nach der Applikation wird der Versuch ausgewertet. Es werden dabei der Neuzuwachs im Vergleich zur Kontrolle bonitiert und gewogen und die Phytotoxizität bewertet. In diesem Versuch zeigen die mit den Wirkstoffen aus der Tabelle 1 behandelten Pflanzen eine deutliche Reduktion des Neuzuwachses ohne dass dabei die Versuchspflanzen geschädigt werden.

Beispiel B4: Wuchsregulierung bei Sojabohnen

Die Pflanzen (z.B. der Sorte Williams) werden in 11 cm - Tontöpfen mit Landerde (45 %), Torf (45 %) und Zonolit (10 %) angesät und in der Klimakammer bei einer Tagestemperatur von 24° C und einer Nachttemperatur von 19° C angezogen. Die Beleuchtungsdauer ist 16 Stunden pro Tag bei einer Intensität von ca. 2900 Lux.

Ca. 24 Tage nach der Saat erfolgt das Umtopfen in 18 cm - Töpfe, 2 Pflanzen/Topf. Nach weiteren 12 Tagen und im Stadium von 5 - 6 trifol. Blätter findet die Applikation mit bis zu 100 g Wirkstoff/ha statt, in der Regel 25 %ig formuliert und in wässriger Spritzbrühe. Die Wasseraufwandmenge beträgt ca. 200 l/ha.

Ca. 4 Wochen nach der Applikation findet die Auswertung statt. Im Vergleich zu unbehandelten Kontrollpflanzen bewirken die erfindungsgemässen Wirkstoffe aus der Tabelle 1 eine merkliche Wuchshemmung ohne Phytotoxizität.

Beispiel B5: Selektive Wuchshemmung von Raps und Klee in Mais

Die Verbindungen der Tabelle 1 sind sehr gut dazu geeignet, bodendeckende Pflanzen in einer Maiskultur selektiv stark zu hemmen und so die Wasser- und Nährstoffkonkurrenz der Untersaat auszuschalten bei gleichzeitiger Aufrechterhaltung der agronomisch erwünschten Wirkungen der Untersaat wie Erosionsschutz, Stickstoffbindung und Reduzierung der Bodenverdichtung. Dies sei am Beispiel der Verbindung Nr. 1.01 für die Kulturkombinationen Mais/Weissklee und Mais/Raps in Tabelle 4 gezeigt:

Mais 'Blizzard' wird in 15 cm-Töpfen mit Landerde ausgesät und im Gewächshaus bei Tag-/Nachttemperaturen von 22/19° C und einer Beleuchtungsdauer von mindestens 13.5 Stunden/Tag für die postemergente Applikation 15 Tage kultiviert, die preemergente Applikation erfolgt einen Tag nach der Saat.

Raps 'Bienvenue' wird in 15 cm-Töpfen mit Landerde ausgesät, 7 Tage bei Tag-/Nachttemperaturen von 22/19° C, dann 17 Tage bei 10/5° C, dann 7 Tage bei 15/10° C bis zur Applikation kultiviert.

Weissklee 'Ladino' wird in 15 cm-Töpfen mit einer Mischung aus Landerde (60 %) Torfsubstrat (30%) und Zonolit (10 %) 40 Tage bei Tag-/Nachttemperaturen von 21/18° C und einer Mindestbeleuchtungsdauer von 13.5 Stunden angezogen.

Düngung und Bewässerung erfolgt in allen Kulturen nach Bedarf, die Applikation der Verbindung Nr. 1.01 (25%ig formuliert) erfolgt in 200 l Wasser/ha und Aufwandmengen von 30 bis 500 g/ha. 28 Tage nach Applikation wird die Höhe des Neuzuwachses gemessen und die Wirkung in Prozent Wuchshemmung im Vergleich zur unbehandelten Kontrolle dargestellt. 100% Wirkung bedeutet völliger Wachstumsstopp, 0 % Wirkung bedeutet Wachstum wie unbehandelte Kontrolle.

Es wurden folgende Ergebnisse erhalten:

Tabelle 4:

| Dosierung g/ha | Mais | | Raps postem. % | Weissklee postem. % |
|---|---|---|---|---|
| | preem. % | postem. | | |
| Kontrolle | 0 | 0 | 0 | 0 |
| 30 | - | 0 | - | - |
| 60 | 0 | 0 | 59 | 25 |
| 125 | 0 | 0 | 76 | 55 |
| 250 | 0 | 0 | 100 | 85 |
| 500 | 0 | 0 | 100 | 100 |

Die Ergebnisse zeigen, dass mit den Verbindungen der Tabelle 1 dikotyle Untersaaten in Mais selektiv gehemmt werden können. Die Applikation kann, bezogen auf Mais, pre-oder postemergent erfolgen.

**Ansprüche**

1. 1-(3,5-Bis-trifluormethylphenyl)-2-thioxo-pyrrolidin-4-carbonsäurederivate der Formel I

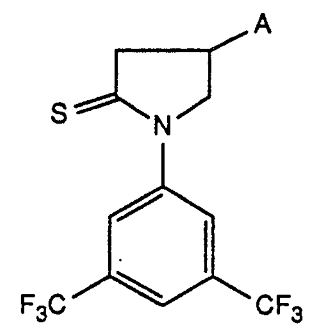

(I)

worin

A für $-COOR_1$, $-COSR_1$, $-COO^{\ominus}M^{\oplus}$, $-CONR_2R_3$ oder -COCl;

$R_1$ für Wasserstoff, $C_1$-$C_4$-Alkyl, $C_2$-$C_6$-Alkenyl oder $C_2$-$C_6$-Alkinyl;

$R_2$ und $R_3$ unabhängig voneinander für Wasserstoff, $C_1$-$C_4$-Alkyl oder $C_3$-$C_7$-Cycloalkyl;

oder $R_2$ und $R_3$ zusammen mit dem Stickstoffatom, an welches sie gebunden sind, für einen gegebenenfalls bis zu dreifach durch $C_1$-$C_4$-Alkyl substituierten gesättigten 3- bis 7-gliedrigen Heterocyclus, der noch ein weiteres Heteroatom ausgewählt aus der Gruppe O, N und S enthalten kann, stehen; und $M^{\oplus}$ das Aequivalent eines Alkali- oder Erdalkalimetallkations oder $HN^{\oplus}(R_2)_3$ bedeutet, sowie deren Isomere in optisch reiner oder angereicherter Form.

2. Verbindungen der Formel I gemäss Anspruch 1, dadurch gekennzeichnet, dass A für $-COOR_1$ oder $-COO^{\ominus}M^{\oplus}$ steht.

3. Verbindungen gemäss Anspruch 1 der Formel (R)-I

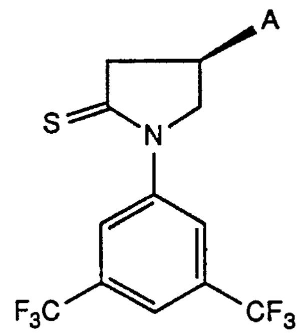

(R)-(I)

worin A die in Anspruch 1 angegebene Bedeutung hat, in optisch reiner oder angereicherter Form.

4. Verbindungen gemäss Anspruch 1 der Formel (S)-I

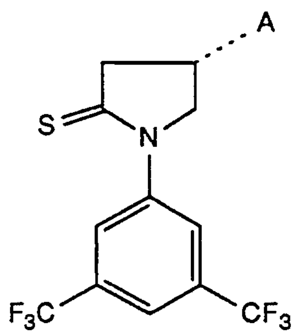

(S)-(I)

worin A die unter Formel I angegebene Bedeutung hat, in optisch reiner oder angereichter Form.

5. Verbindungen der Formel (R)-I gemäss Anspruch 3, dadurch gekennzeichnet, dass A für $-COOR_1$ oder $-COO^{\ominus}M^{\oplus}$ steht.

6. Verbindungen der Formel (S)-I gemäss Anspruch 4, dadurch gekennzeichnet, dass A für $-COOR_1$ oder $-COO^{\ominus}M^{\oplus}$ steht.

7. 4-(R)-(-)-1-(3,5-Bis-trifluormethylphenyl)-2-thioxo-pyrrolidin-4-carbonsäure gemäss Anspruch 1.

8. 4-(R)-(-)-1-(3,5-Bis-trifluormethylphenyl)-2-thioxo-pyrrolidin-4-carbonsäuremethylester gemäss Anspruch 1.

9. 4-(S)-(+)-1-(3,5-Bis-trifluormethylphenyl)-2-thioxo-pyrrolidin-4-carbonsäure gemäss Anspruch 1.

10. 4-(S)-(+)-1-(3,5-Bis-trifluormethylphenyl)-2-thioxo-pyrrolidin-4-carbonsäuremethylester gemäss Anspruch 1.

11. 1-(3,5-Bis-trifluormethylphenyl)-2-thioxo-pyrrolidin-4-carbonsäure gemäss Anspruch 1.

12. 1-(3,5-Bis-trifluormethylphenyl)-2-thioxo-pyrrolidin-4-carbonsäuremethylester gemäss Anspruch 1.

13. Verfahren zur Herstellung der racemischen Verbindungen der Formel I gemäss Anspruch 1, dadurch gekennzeichnet, dass man ein racemisches 1-(3,5-Bis-trifluormethylphenyl)-2-oxo-pyrrolidin der Formel II

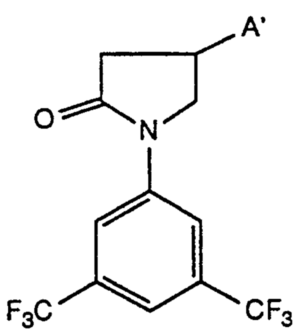

(II)

worin A' für $-COOR_1$ und $R_1$ für $C_1$-$C_4$-Alkyl steht, mit einem zur Ein- führung der Thioxogruppe befähigten Reagenz zu den entsprechenden racemischen 1-(3,5-Bis-trifluormethylphenyl)-2-thioxo-pyrrolidin-4-carbonsäureestern der Formel Ia

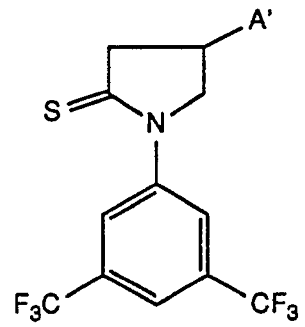

(Ia)

worin $A'$ für $-COOR_1$ und $R_1$ für $C_1$-$C_4$-Alkyl steht, umsetzt, und diese Verbindungen gegebenenfalls anschliessend auf an sich bekannte Weise in die anderen racemischen definitionsgemässen Derivate der Formel I, worin A die unter Formel I angegebene Bedeutung hat, überführt.

14. Verfahren zur Herstellung der Verbindungen der Formeln (R)-I und (S)-I gemäss den Ansprüchen 3 und 4, dadurch gekennzeichnet, dass man die racemische 1-(3,5-Bis-trifluormethylphenyl)-2-oxo-pyrrolidin-4-carbonsäure der Formel IIa

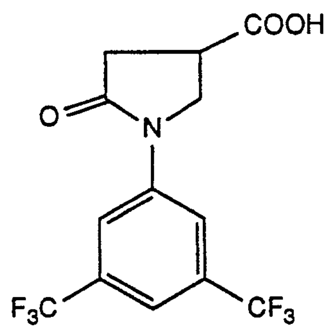

(IIa)

durch die an sich bekannte Methode der fraktionierten Kristallisation in Gegenwart eines chiralen Hilfsstoffes in die optisch aktiven Isomere der Formel (R)-IIa und (S)-IIa

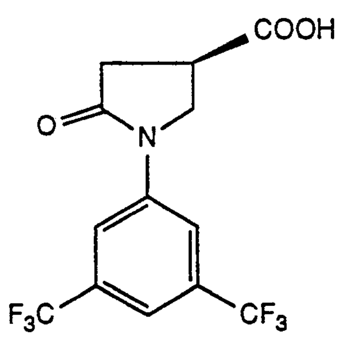

(R)-(IIa)

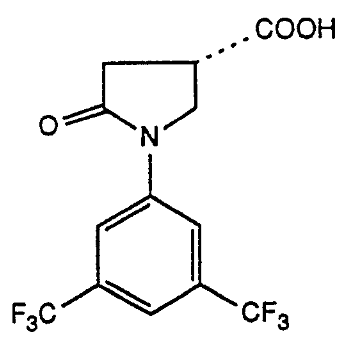

(S)-(IIa)

auftrennt, diese gegebenenfalls auf an sich bekannte Weise in die isomeren Derivate der Formel (R)-IIb und (S)-IIb

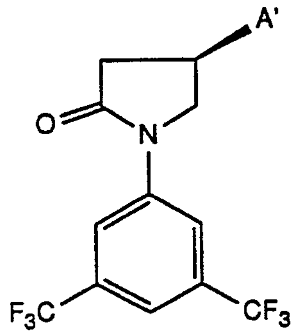
(R)-(IIb)

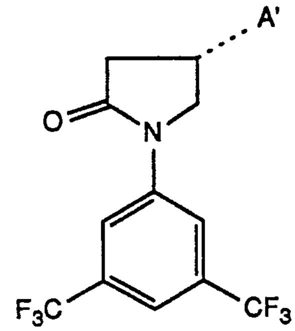
(S)-(IIb)

worin A′ für -$COOR_1$ und $R_1$ für $C_1$-$C_4$-Alkyl steht, überführt, um anschliessend durch Umsetzung mit einem zur Einführung der Thioxogruppe befähigten Reagenz die Verbindungen der Formeln (R)-Ia und (S)-Ia

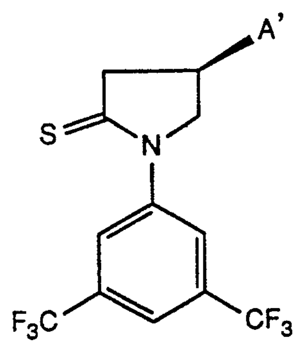
(R)-(Ia)

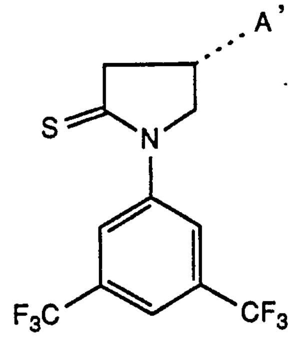
(S)-(Ia)

worin
A′ für -$COOR_1$ und $R_1$ für $C_1$-$C_4$-Alkyl steht, zu erhalten, welche gegebenenfalls auf an sich bekannte Weise in die übrigen definitionsgemässen Derivate der Formeln (R)-I und (S)-I überführt werden können.

15. Verfahren gemäss einem der Ansprüche 13 oder 14, dadurch gekennzeichnet, dass man als zur Einführung der Thioxogruppe befähigtes Reagenz 2,4-Bis-(4-methoxyphenyl)-1,3-dithia-2,4-diphosphetan-2,4-disulfid verwendet.

16. Verbindungen der Formeln

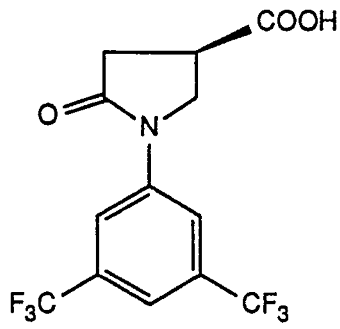

(R)-(IIa)

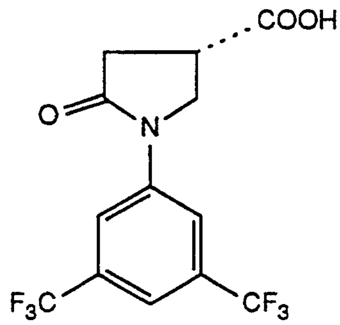

(S)-(IIa)

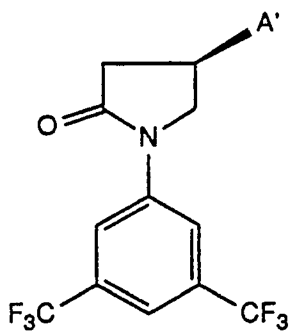

(R)-(IIb)

oder

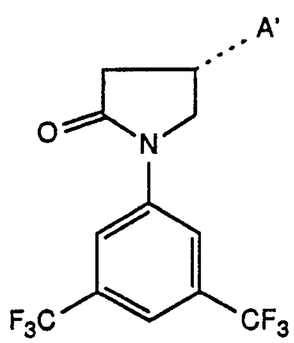

(S)-(IIb)

worin A' für $-COOR_1$ und $R_1$ für $C_1$-$C_4$-Alkyl steht, in optisch reiner oder angereicherter Form.

17. Die Verwendung von 1-(3,5-Bis-trifluormethylphenyl)-2-thioxo-pyrrolidin-4-carbonsäurederivaten der Formel I

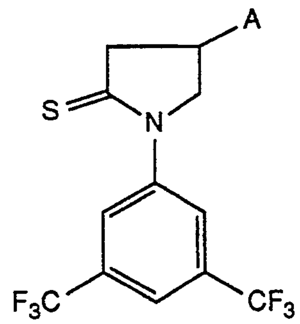

(I)

worin A die unter Anspruch 1 angegebene Bedeutung hat, zum Schützen von Kulturpflanzen gegen die schädigende Wirkung von Herbiziden.

18. Die Verwendung gemäss Anspruch 17, dadurch gekennzeichnet, dass A die unter Anspruch 2 angegebene Bedeutung hat.

19. Die Verwendung gemäss Anspruch 17 oder 18 gegen die schädigende Wirkung von Chloracetanilidherbiziden oder Sulfonylharnstoffherbiziden.

20. Die Verwendung gemäss Anspruch 19 in Kulturen von Mais, Sorghum und Reis.

21. Die Verwendung gemäss Anspruch 17 oder 18 gegen die schädigende Wirkung herbizider Sulfonylharnstoffderivate der Formel III

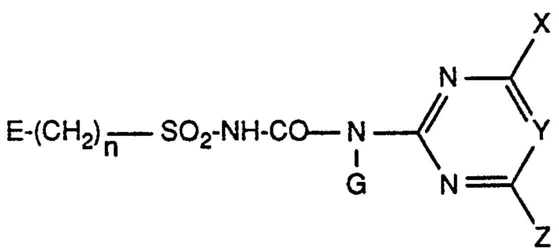

(III)

worin

E eine Gruppe

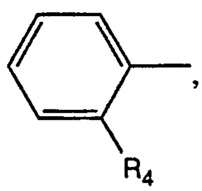, 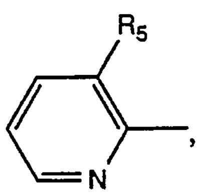, 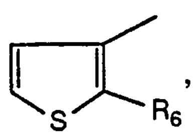, oder 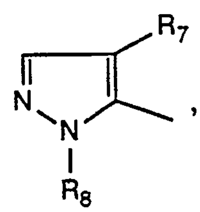,

n die Zahl null oder eins,
G Wasserstoff oder Methyl,
X Methoxy, Aethoxy, Difluormethoxy, Methyl oder Chlor
Y CH oder N,
Z Methoxy, Methyl, Difluormethoxy, Cyclopropyl oder Methylamino,
$R_4$ $C_2$-$C_5$-Alkoxyalkoxy,. $C_1$-$C_4$-Halogenalkoxy, $C_1$-$C_4$-Halogenalkylthio, $C_2$-$C_4$-Halogenalkenyl, Chlor oder $C_1$-$C_4$-Alkoxycarbonyl,
$R_5$ Trifluormethyl oder Di($C_1$-$C_4$-alkyl)carbamoyl,
$R_6$ $C_1$-$C_4$-Alkoxycarbonyl,
$R_7$ $C_1$-$C_4$-Alkoxycarbonyl, und
$R_8$ $C_1$-$C_4$-Alkyl bedeuten.

22. Die Verwendung gemäss Anspruch 21 gegen die schädigende Wirkung von
N-(3-Trifluormethylpyridin-2-ylsulfonyl)-N'-(4,6-dimethoxypy rimidin-2-yl)-harnstoff,
N-(3-Dimethylcarbamoylpyridin-2-ylsulfonyl)-N'-(4,6-dimethoxyprimidin-2-yl)-harnstoff,
N-(1-Methyl-4-ethoxycarbonylpyrazol-2-ylsulfonyl)-N'-(4,6-dimethoxy-yrimidin-2-yl)-harnstoff,

N-(2-Methoxycarbonylthien-3-ylsulfonyl)-N′-(4-methoxy-6-methyl- 1,3,5-triazin-2-yl)-harnstoff,
N-(2-Methoxycarbonylbenzylsulfonyl)-N′-(4,6-dimethoxypyrimidin-2-yl)-harnstoff,
N-(2-Methoxycarbonylphenylsulfonyl)-N′-(4,6-bis-difluormethoxy-pyrimidin-2-yl)-harnstoff,
N-(2-Methoxycarbonylphenylsulfonyl)-N′-(4-ethoxy-6-methylamino- 1,3,5-triazin-2-yl)-harnstoff,
N-(2-Methoxycarbonylphenylsulfonyl)-N′-(4-methoxy-6-methyl-1,3,5-triazin-2-yl)-harnstoff,
N-(2-Ethoxycarbonylphenylsulfonyl)-N′-(4-chlor-6-methoxypyrimidin-2-yl)-harnstoff,
N-(2-Methoxycarbonylphenylsulfonyl)-N′-(4-methoxy-6-methyl-1,3,5-triazin-2-yl)-N′-methylharnstoff,
N-(2-Methoxycarbonylphenylsulfonyl)-N′-(4,6-dimethoxypyrimidin-2-yl)-harnstoff,
N-(2-Chlorphenylsulfonyl)-N′-(4-methoxy-6-methyl-1,3,5-triazin-2-yl)-harnstoff,
N-[2-(2-Chlorethoxy)-phenylsulfonyl]-N′-(4-methoxy-6-methyl-1,3,5-triazin-2-yl)-harnstoff, und
N-[2-(2-Methoxyethoxy)-phenylsulfonyl]-N′-(4,6-dimethoxy-1,3,5-triazin-2-yl)-harnstoff.

23. Die Verwendung gemäss Anspruch 17 oder 18 gegen die schädigende Wirkung herbizider Halogenacetanilide der Formel IV

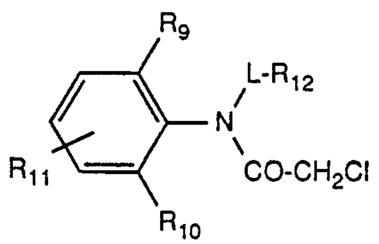

(IV)

worin
L eine $C_1$-$C_4$-Alkylenbrücke,
$R_9$, $R_{10}$ und $R_{11}$ unabhängig voneinander Wasserstoff, Halogen, $C_1$-$C_4$-Alkyl,
$C_1$-$C_4$-Alkoxy, $C_1$-$C_4$-Halogenalkyl, $C_2$-$C_5$-Alkoxyalkyl oder $C_2$-$C_5$-Alkylthioalkyl, und
$R_{12}$ $C_1$-$C_4$-Alkoxy, -COOH, $C_1$-$C_4$-Alkoxycarbonyl, -$CONH_2$, Wasserstoff, $C_1$-$C_4$-Alkylcarbamoyl, Di-$C_1$-$C_4$-alkylcarbamoyl, Cyan, $C_1$-$C_4$-Alkylcarbonyl,
gegebenenfalls substituiertes Benzoyl,
gegebenenfalls substituiertes Furyl,
gegebenenfalls substituiertes Thienyl,
gegebenenfalls substituiertes Pyrrolyl,
gegebenenfalls substituiertes Pyrazolyl,
gegebenenfalls substituiertes 1,3,4-Oxadiazol-2-yl,
gegebenenfalls substituiertes 1,3,4-Thiadiazol-2-yl,
gegebenenfalls substituiertes Dioxolanyl,
gegebenenfalls substituiertes Dioxanyl,
gegebenenfalls substituiertes 1,3,4-Triazol-2-yl oder
gegebenenfalls substituiertes Tetrahydrofuryl, bedeutet.

24. Die Verwendung gemäss Anspruch 23 gegen die schädigende Wirkung herbizider Halogenacetanilide der Formel IV

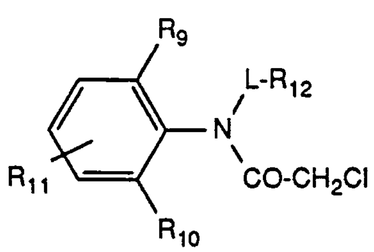

(IV)

worin
L eine $C_1$-$C_4$-Alkylenbrücke,
$R_9$, $R_{10}$ und $R_{11}$ unabhängig voneinander Wasserstoff, Halogen, $C_1$-$C_4$-Alkyl, $C_1$-$C_4$-Alkoxy, $C_1$-$C_4$-Halogenalkyl, $C_2$-$C_5$-Alkoxyalkyl oder $C_2$-$C_5$-Alkylthioalkyl, und
$R_{12}$ $C_1$-$C_4$-Alkoxy, -COOH, $C_1$-$C_4$-Alkoxycarbonyl, -$CONH_2$, Wasserstoff, $C_1$-$C_4$-Alkylcarbamoyl, Di-$C_1$-$C_4$-

alkylcarbamoyl, Cyan, $C_1$-$C_4$-Alkylcarbonyl, bedeutet.

25. Die Verwendung gemäss Anspruch 23 gegen die schädigende Wirkung von
N-Aethoxymethyl-N-chloracetyl-2-ethyl-6-methylanilin,
N-Chloracetyl-N-methoxymethyl-2,6-diethylanilin,
N-Chloracetyl-N-(2-methoxyethyl)-2,6-dimethylanilin,
N-Chloracetyl-N-(2-n-propoxyethyl)-2,6-dimethylanilin,
N-Chloracetyl-N-(2-isopropoxyethyl)-2,6-dimethylanilin,
N-Chloracetyl-N-(2-methoxyethyl)-2-ethyl-6-methylanilin,
N-Chloracetyl-N-(methoxyethyl)-2,6-diethylanilin,
N-(2-Ethoxyethyl)-N-chloracetyl-2-ethyl-6-methylanilin,
N-Chloracetyl-N-(2-methoxy-1-methylethyl)-2-methylanilin,
N-Chloracetyl-N-(2-methoxy-1-methylethyl)-2,6-dimethylanilin,
N-Chloracetyl-N-(2-methoxy-1-methylethyl)-2,6-diethylanilin,
N-Chloracetyl-N-(2-methoxy-1-methylethyl)-2-ethyl-6-methylani lin,
N-(2-Ethoxyethyl)-N-chloracetyl-2,6-diethylanilin,
N-Chloracetyl-N-(2-n-propoxyethyl)-2-ethyl-6-methylanilin,
N-Chloracetyl-N-(2-n-propoxyethyl)-2,6-diethylanilin,
N-Chloracetyl-N-(2-isopropoxyethyl)-2-ethyl-6-methylanilin,
N-Ethoxycarbonylmethyl-N-chloracetyl-2,6-dimethylanilin,
N-Ethoxycarbonylmethyl-N-chloracetyl-2,6-diethylanilin,
N-Chloracetyl-N-methoxycarbonylmethyl-2,6-dimethylanilin,
N-Chloracetyl-N-(2,2-diethoxyethyl)-2,6-dimethylanilin,
N-Chloracetyl-N-(2-methoxy-1-methylethyl)-2,3-dimethylanilin,
N-(2-Ethoxyethyl)-N-chloracetyl-2-methylanilin,
N-Chloracetyl-N-(2-methoxyethyl)-2-methylanilin,
N-Chloracetyl-N-(2-methoxy-2-methylethyl)-2,6-dimethylanilin,
N-(2-Ethoxy-2-methylethyl)-N-chloracetyl-2-ethyl-6-methylanilin,
N-Chloracetyl-N-(1-ethyl-1-methoxyethyl)-2,6-dimethylanilin,
N-Chloracetyl-N-(2-methoxyethyl)-2-methoxy-6-methylanilin,
N-n-Butoxymethyl-N-chloracetyl-2-tert.-butylanilin,
N-Chloracetyl-N-(2-ethoxyethyl-2-methylethyl)-2,6-dimethylanilin,
N-Chloracetyl-N-(-2-methoxyethyl)-2-chlor-6-methylanilin,
N-(2-Ethoxyethyl)-N-chloracetyl-2-chlor-6-methylanilin,
N-(2-Ethoxyethyl)-N-chloracetyl-2,3,6-trimethylanilin,
N-Chloracetyl-1-(2-methoxyethyl)-2,3,6-trimethylanilin,
N-Chloracetyl-N-cyanomethyl-2,6-dimethylanilin,
N-Chloracetyl-N-(1,3-dioxolan-2-ylmethyl)-2,6-dimethylanilin,
N-Chloracetyl-N-(1,3-dioxolan-2-ylmethyl)-2-ethyl-6-methylanilin,
N-Chloracetyl-N-(1,3-dioxan-2-ylmethyl)-2-ethyl-6-methylanilin,
N-Chloracetyl-N-(2-furylmethyl)-2,6-dimethylanilin,
N-Chloracetyl-N-(2-furylmethyl)-2-ethyl-6-methylanilin,
N-Chloracetyl-N-(2-tetrahydrofurylmethyl)-2,6-dimethylanilin,
N-Chloracetyl-N-(N,N-dimethylcarbamoylmethyl)-2,6-dimethylanilin,
N-(n-Butoxymethyl)-N-chloracetyl-2,6-diethylanilin,
N-(2-n-Butoxyethyl)-N-chloracetyl-2,6-diethylanilin,
N-Chloracetyl-N-(2-methoxy- 1,2-dimethylethyl)-2,6-dimethylanilin,
N-Chloracetyl-N-isopropyl-2,3-dimethylanilin,
N-Chloracetyl-N-isopropyl-2-chloranilin,
N-Chloracetyl-N-(1H-pyrazol-1-ylmethyl)-2,6-dimethylanilin,
N-Chloracetyl-N-(1H-pyrazol-1-ylmethyl)-2-ethyl-6-methylanilin,
N-Chloracetyl-N-(1H-1,2,4-triazol-1-ylmethyl)-2,6-dimethylanilin,
N-Chloracetyl-N-(1H-1,2,4-triazol-1-ylmethyl)-2,6-diethylanilin,
N-Benzoylmethyl-N-chloracetyl-2,6-diethylanilin,
N-Benzoylmethyl-N-chloracetyl-2-ethyl-6-methylanilin,
N-Chloracetyl-N-(5-methyl-1,3,4-oxadiazol-2-yl)-2,6-diethylanilin,
N-Chloracetyl-N-(5-methyl-1,3,4-oxadiazol-2-yl)-2-ethyl-6-methylanilin,
N-Chloracetyl-N-(5-methyl-1,3,4-oxadiazol-2-yl)-2-tert.butylanilin,
N-Chloracetyl-N-(4-chlorbenzoylmethyl)-2,6-dimethylanilin und

N-Chloracetyl-N-(1-methyl-5-methylthio-1,3,4-triazol-2-ylmethyl)-2,6-diethylanilin.

26. Die Verwendung gemäss Anspruch 17 oder 18 gegen die schädigende Wirkung von N-Chloracetyl-N-(2-n-propoxyethyl)-2,6-diethylanilin.

27. Verfahren zum Schützen von Kulturpflanzen gegen die kulturpflanzenschädigende Wirkung von Herbiziden dadurch gekennzeichnet, dass man vor, während oder nach der Applikation des Herbizids die Kulturpflanze, deren Lebensraum, Teile der Kulturpflanze oder deren Samen oder Stecklinge der Kulturpflanze mit einer antagonistisch wirksamen Menge von 1-(3,5-Bis-trifluormethylphenyl)-2-thioxo-pyrrolidin-4-carbonsäurederivaten der Formel I

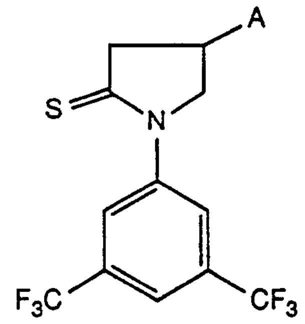

(I)

worin A die unter Anspruch 1 angegebene Bedeutung hat, behandelt.

28. Verfahren nach Anspruch 26, dadurch gekennzeichnet, dass A die unter Anspruch 2 angegebene Bedeutung hat.

29. Verfahren nach Anspruch 26 oder 27 zum Schützen von Kulturpflanzen gegen die schädigende Wirkung von Chloracetanilidherbiziden oder Sulfonylharnstoffherbiziden.

30. Verfahren nach Anspruch 28 zum Schützen von Reis, Sorghum und Mais.

31. Herbizide Mittel enthaltend ein Herbizid und eine die kulturpflanzenschädigende Wirkung antagonisierende Menge von 1-(3,5-Bis-trifluormethyl-phenyl)-2-thioxo-pyrrolidin-4-carbonsäurederivaten der Formel I,

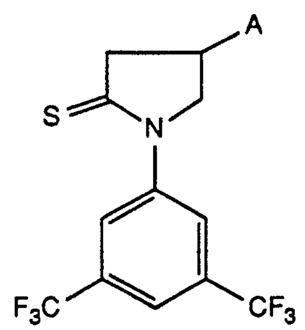

(I)

worin A die unter Anspruch 1 angegebene Bedeutung hat, sowie gewünschtenfalls weitere Hilfs- und/oder Trägerstoffe.

32. Herbizides Mittel nach Anspruch 31, dadurch gekennzeichnet, dass A die unter Anspruch 2 angegebene Bedeutung hat.

33. Herbizides Mittel gemäss Anspruch 31 oder 32 enthaltend ein Herbizid aus der Gruppe der Chloracetanilide oder Sulfonylharnstoffe.

34. Saatgut von Kulturpflanzen, welches mit einer antagonistisch wirksamen Menge von 1-(3,5-Bis-trifluormethylphenyl)-2-thioxo-pyrrolidin-4-carbonsäurederivaten der Formel I,

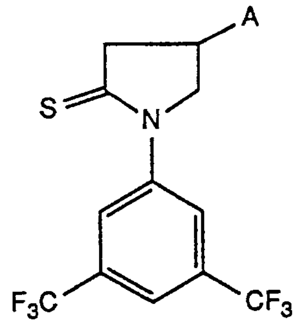

(I)

worin A die unter Anspruch 1 angegebene Bedeutung hat, behandelt worden ist.

35. Saatgut nach Anspruch 34, dadurch gekennzeichnet, dass A die unter Anspruch 2 angegebene Bedeutung hat.

36. Das Saatgut von Sorghum, Reis oder Mais gemäss den Ansprüchen 34 oder 35.

37. Ein Pflanzenwuchs hemmendes Mittel, dadurch gekennzeichnet, dass es neben Träger- und/oder anderen Zuschlagstoffen als Wirkstoff mindestens ein Pyrrolidincarbonsäurederivat der Formel I, gemäss einem der Ansprüche 1 bis 12, enthält.

38. Mittel gemäss Anspruch 37, dadurch gekennzeichnet, dass es zwischen 0,1 % und 95 % Wirkstoff der Formel I gemäss einem der Ansprüche 1 bis 12 enthält.

39. Verfahren zur Hemmung des Pflanzenwachstums, dadurch gekennzeichnet, dass man einen Wirkstoff der Formel I, gemäss einem der Ansprüche 1 bis 12, oder ein diesen Wirkstoff enthaltendes Mittel gemäss Anspruch 37 in einer wirksamen Menge auf die Pflanzen oder deren Lebensraum appliziert.

40. Verfahren gemäss Anspruch 39, dadurch gekennzeichnet, dass man eine Wirkstoffmenge zwischen 0,01 und 10 kg pro Hektar appliziert.

41. Verfahren zur Beeinflussung des Pflanzenwachstums zum Zweck der Ertragssteigerung, dadurch gekennzeichnet, dass man einen Wirkstoff der Formel I, gemäss einem der Ansprüche 1 bis 12, oder ein diesen Wirkstoff enthaltendes Mittel gemäss Anspruch 37 in einer wirksamen Menge auf die Pflanzen oder deren Lebensraum appliziert.

Patentansprüche für folgenden Vertragsstaat: ES

1. Herbizide Mittel enthaltend ein Herbizid und eine die kulturpflanzenschädigende Wirkung antagonisierende Menge von 1-(3,5-Bis-trifluormethyl-phenyl)-2-thioxo-pyrrolidin-4-carbonsäurederivaten der Formel I,

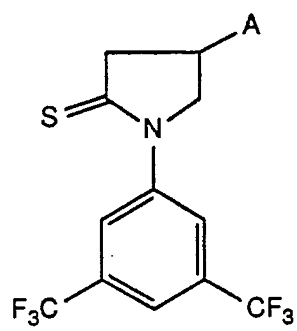

(I)

worin
A für $-COOR_1$, $-COSR_1$, $-COO^{\ominus}M^{\oplus}$, $-CONR_2R_3$ oder -COCl;
$R_1$ für Wasserstoff, $C_1$-$C_4$-Alkyl, $C_2$-$C_6$-Alkenyl oder $C_2$-$C_6$-Alkinyl;
$R_2$ und $R_3$ unabhängig voneinander für Wasserstoff, $C_1$-$C_4$-Alkyl oder $C_3$-$C_7$-Cycloalkyl;
oder $R_2$ und $R_3$ zusammen mit dem Stickstoffatom, an welches sie gebunden sind, für einen gegebenenfalls bis zu dreifach durch $C_1$-$C_4$-Alkyl substituierten gesättigten 3- bis 7-gliedrigen Heterocyclus, der noch ein weiteres Heteroatom ausgewählt aus der Gruppe O, N und S enthalten kann, stehen; und $M^{\oplus}$ das Aequivalent eines Alkali- oder Erdalkalimetallkations oder $HN^{\oplus}(R_2)_3$ bedeutet, sowie deren Isomere in optisch reiner oder angereicherter Form.

2. Herbizides Mittel gemäss Anspruch 1, dadurch gekennzeichnet, dass A für $-COOR_1$ oder $-COO^{\ominus}M^{\oplus}$ steht.

3. Herbizides Mittel gemäss Anspruch 1, dadurch gekennzeichnet, dass es Verbindungen gemäss Anspruch 1 der Formel (R)-I

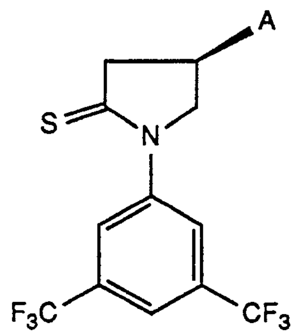

(R)-(I)

worin A die in Anspruch 1 angegebene Bedeutung hat, in optisch reiner oder angereicherter Form, enthält.

4. Herbizides Mittel gemäss Anspruch 1, dadurch gekennzeichnet, dass es Verbindungen gemäss Anspruch 1 der Formel (S)-I

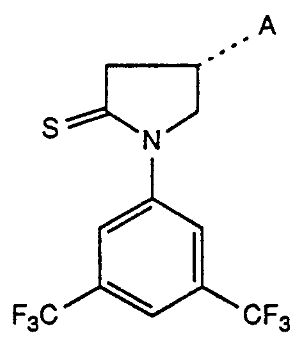

(S)-(I)

worin A die unter Formel I angegebene Bedeutung hat, in optisch reiner oder angereichter Form, enthält.

5. Herbizides Mittel gemäss Anspruch 3, dadurch gekennzeichnet, dass A für $-COOR_1$ oder $-COO^{\ominus}M^{\oplus}$ steht.

6. Herbizides Mittel gemäss Anspruch 4, dadurch gekennzeichnet, dass A für $-COOR_1$ oder $COO^{\ominus}M^{\oplus}$ steht.

7. Herbizides Mittel gemäss Anspruch 1, dadurch gekennzeichnet, dass es als Verbindungen der Formel I 4-(R)-(-)-1-(3,5-Bis-trifluormethylphenyl)-2-thioxo-pyrrolidin-4-carbonsäure enthält.

8. Herbizides Mittel gemäss Anspruch 1, dadurch gekennzeichnet, dass es als Verbindun gen der Formel I 4-(R)-(-)-1-(3,5-Bis-trifluormethylphenyl)-2-thioxo-pyrrolidin-4-carbonsäuremethylester enthält.

9. Herbizides Mittel gemäss Anspruch 1, dadurch gekennzeichnet, dass es als Verbindungen der Formel I 4-(S)-(+)-1-(3,5-Bis-trifluormethylphenyl)-2-thioxo-pyrrolidin-4-carbonsäure enthält.

10. Herbizides Mittel gemäss Anspruch 1, dadurch gekennzeichnet, dass es als Verbindungen der Formel I 4-(S)-(+)-1-(3,5-Bis-trifluormethylphenyl)-2-thioxo-pyrrolidin-4-carbonsäuremethylester enthält

11. Herbizides Mittel gemäss Anspruch 1, dadurch gekennzeichnet, dass es als Verbindungen der Formel I 1-(3-5-Bis-trifluormethylphenyl)-2-thioxo-pyrrolidin-4-carbonsäure enthält

12. Herbizides Mittel gemäss Anspruch 1, dadurch gekennzeichnet, dass es als Verbindungen der Formel I 1-(3,5-Bis-trifluormethylphenyl)-2-thioxo-pyrrolidin-4-carbonsäuremethylester enthält

13. Verfahren zur Herstellung der racemischen Verbindungen der Formel I gemäss Anspruch 1, dadurch gekennzeichnet, dass man ein racemisches 1-(3,5-Bis-trifluormethylphenyl)-2-oxo-pyrrolidin der Formel II

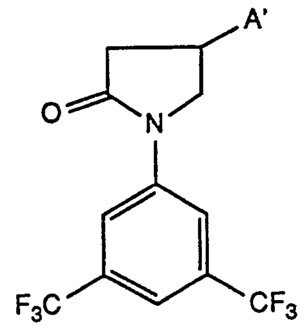

(II)

worin A′ für -COOR₁ und R₁ für C₁-C₄-Alkyl steht, mit einem zur Ein- führung der Thioxogruppe befähigten Reagenz zu den entsprechenden racemischen 1-(3,5-Bis-trifluormethylphenyl)-2-thioxo-pyrrolidin-4-carbonsäureestern der Formel Ia

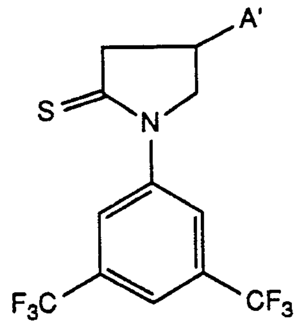

(Ia)

worin A′ für -COOR₁ und R₁ für $C_1$-$C_4$-Alkyl steht, umsetzt, und diese Verbindungen gegebenenfalls anschliessend auf an sich bekannte Weise in die anderen racemischen definitionsgemässen Derivate der Formel I, worin A die unter Formel I angegebene Bedeutung hat, überführt.

14. Verfahren zur Herstellung der Verbindungen der Formeln (R)-I und (S)-I gemäss den Ansprüchen 3 und 4, dadurch gekennzeichnet, dass man die racemische 1-(3,5-Bis-trifluormethylphenyl)-2-oxo-pyrrolidin-4-carbonsäure der Formel IIa

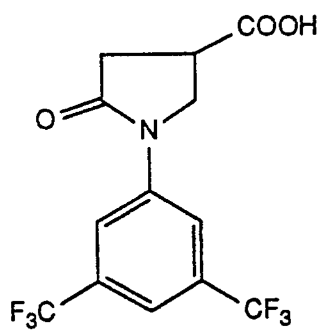

(IIa)

durch die an sich bekannte Methode der fraktionierten Kristallisation in Gegenwart eines chiralen Hilfsstoffes in die optisch aktiven Isomere der Formel (R)-IIa und (S)-IIa

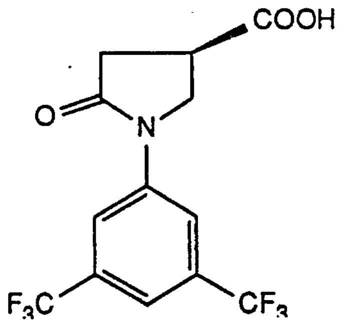 (R)-(IIa) 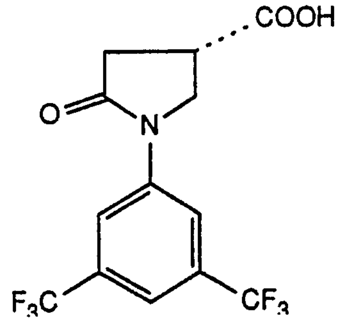 (S)-(IIa)

auftrennt, diese gegebenenfalls auf an sich bekannte Weise in die isomeren Derivate der Formel (R)-IIb und (S)-IIb

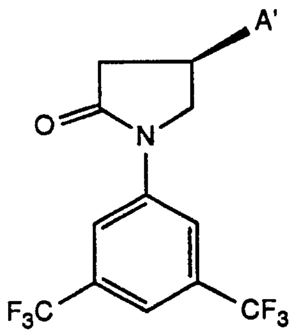 (R)-(IIb) 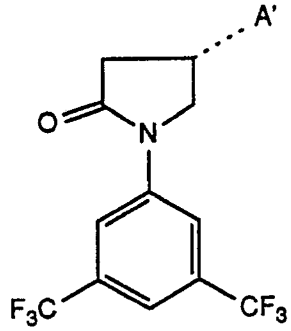 (S)-(IIb)

worin A′ für -COOR$_1$ und R$_1$ für C$_1$-C$_4$-Alkyl steht, überführt, um anschliessend durch Umsetzung mit einem zur Einführung der Thioxogruppe befähigten Reagenz die Verbindungen der Formeln (R)-Ia und (S)-Ia

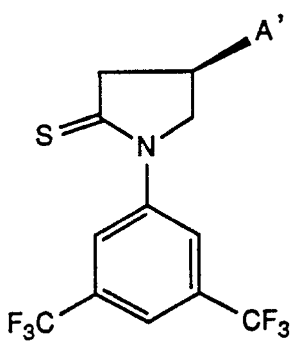 (R)-(Ia) 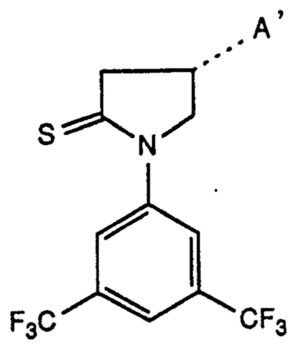 (S)-(Ia)

worin
A' für -COOR$_1$ und R$_1$ für C$_1$-C$_4$-Alkyl steht, zu erhalten, welche gegebenenfalls auf an sich bekannte Weise in die übrigen definitionsgemässen Derivate der Formeln (R)-I und (S)-I überführt werden können.

15. Verfahren gemäss einem der Ansprüche 13 oder 14, dadurch gekennzeichnet, dass man als zur Einführung der Thioxogruppe befähigtes Reagenz 2,4-Bis-(4-methoxyphenyl)-1,3-dithia-2,4-diphosphetan-2,4-disulfid verwendet.

16. Die Verwendung von 1-(3,5-Bis-trifluormethylphenyl)-2-thioxo-pyrrolidin-4-carbonsäurederivaten der Formel I

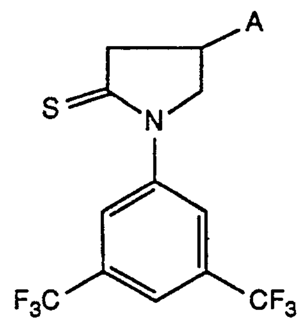

(I)

worin A die unter Anspruch 1 angegebene Bedeutung hat, zum Schützen von Kulturpflanzen gegen die schädigende Wirkung von Herbiziden.

17. Die Verwendung gemäss Anspruch 16, dadurch gekennzeichnet, dass A die unter Anspruch 2 angegebene Bedeutung hat.

18. Die Verwendung gemäss Anspruch 16 oder 17 gegen die schädigende Wirkung von Chloracetanilidherbiziden oder Sulfonylharnstoffherbiziden.

19. Die Verwendung gemäss Anspruch 18 in Kulturen von Mais, Sorghum und Reis.

20. Die Verwendung gemäss Anspruch 16 oder 17 gegen die schädigende Wirkung herbizider Sulfonylharnstoffderivate der Formel III

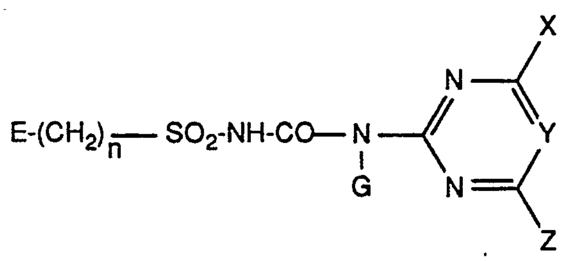

(III)

worin

E eine Gruppe

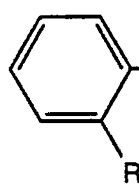, 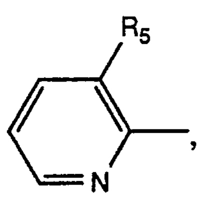, 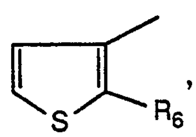, oder 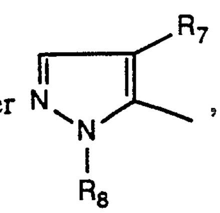,

n die Zahl null oder eins,

G Wasserstoff oder Methyl,

X Methoxy, Aethoxy, Difluormethoxy, Methyl oder Chlor

Y CH oder N,

Z Methoxy, Methyl, Difluormethoxy, Cyclopropyl oder Methylamino,

$R_4$ $C_2$-$C_5$-Alkoxyalkoxy, $C_1$-$C_4$-Halogenalkoxy, $C_1$-$C_4$-Halogenalkylthio, $C_2$-$C_4$-Halogenalkenyl, Chlor oder $C_1$-$C_4$-Alkoxycarbonyl,

$R_5$ Trifluormethyl oder Di($C_1$-$C_4$-alkyl)carbamoyl,

$R_6$ $C_1$-$C_4$-Alkoxycarbonyl,

$R_7$ $C_1$-$C_4$-Alkoxycarbonyl, und

$R_8$ $C_1$-$C_4$-Alkyl bedeuten.

21. Die Verwendung gemäss Anspruch 20 gegen die schädigende Wirkung von
N-(3-Trifluormethylpyridin-2-ylsulfonyl)-N′-(4,6-dimethoxypy rimidin-2-yl)-harnstoff,
N-(3-Dimethylcarbamoylpyridin-2-ylsulfonyl)-N′-(4,6-dimethoxypyrimidin-2-yl)-harnstoff,
N-(1-Methyl-4-ethoxycarbonylpyrazol-2-ylsulfonyl)-N′-(4,6-dimethoxy-yrimidin-2-yl)-harnstoff,

N-(2-Methoxycarbonylthien-3-ylsulfonyl)-N'-(4-methoxy-6-methyl- 1,3,5-triazin-2-yl)-harnstoff,
N-(2-Methoxycarbonylbenzylsulfonyl)-N'-(4,6-dimethoxyprimidin-2-yl)-harnstoff,
N-(2-Methoxycarbonylphenylsulfonyl)-N'-(4,6-bis-difluormethoxy-pyrimidin-2-yl)-harnstoff,
N-(2-Methoxycarbonylphenylsulfonyl)-N'-(4-ethoxy-6-methylamino-1,3,5-triazin-2-yl)-harnstoff,
N-(2-Methoxycarbonylphenylsulfonyl)-N'-(4-methoxy-6-methyl-1,3,5-triazin-2-yl)-harnstoff,
N-(2-Ethoxycarbonylphenylsulfonyl)-N'-(4-chlor-6-methoxypyrimidin-2-yl)-harnstoff,
N-(2-Methoxycarbonylphenylsulfonyl)-N'-(4-methoxy-6-methyl-1,3,5-triazin-2-yl)-N'-methylharnstoff,
N-(2-Methoxycarbonylphenylsulfonyl)-N'-(4,6-dimethoxypyrimidin-2-yl)-harnstoff,
N-(2-Chlorphenylsulfonyl)-N'-(4-methoxy-6-methyl-1,3,5-triazin-2-yl)-harnstoff,
N-[2-(2-Chlorethoxy)-phenylsulfonyl]-N'-(4-methoxy-6-methyl-1,3,5-triazin-2-yl)-harnstoff, und
N-[2-(2-Methoxyethoxy)-phenylsulfonyl]-N'-(4,6-dimethoxy-1,3,5-triazin-2-yl)-harnstoff.

22. Die Verwendung gemäss Anspruch 16 oder 17 gegen die schädigende Wirkung herbizider Halogenacetanilide der Formel IV

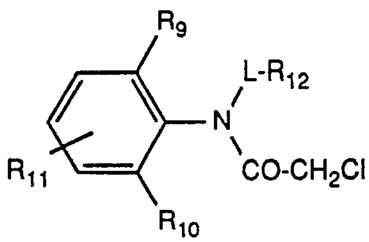

(IV)

worin
L eine $C_1$-$C_4$-Alkylenbrücke,
$R_9$, $R_{10}$ und $R_{11}$ unabhängig voneinander Wasserstoff, Halogen, $C_1$-$C_4$-Alkyl, $C_1$-$C_4$-Alkoxy, $C_1$-$C_4$-Halogenalkyl, $C_2$-$C_5$-Alkoxyalkyl oder $C_2$-$C_5$-Alkylthioalkyl, und
$R_{12}$ $C_1$-$C_4$-Alkoxy, -COOH, $C_1$-$C_4$-Alkoxycarbonyl, -$CONH_2$, Wasserstoff, $C_1$-$C_4$-Alkylcarbamoyl, Di-$C_1$-$C_4$-alkylcarbamoyl, Cyan, $C_1$-$C_4$-Alkylcarbonyl,
gegebenenfalls substituiertes Benzoyl,
gegebenenfalls substituiertes Furyl,
gegebenenfalls substituiertes Thienyl,
gegebenenfalls substituiertes Pyrrolyl,
gegebenenfalls substituiertes Pyrazolyl,
gegebenenfalls substituiertes 1,3,4-Oxadiazol-2-yl,
gegebenenfalls substituiertes 1,3,4-Thiadiazol-2-yl,
gegebenenfalls substituiertes Dioxolanyl,
gegebenenfalls substituiertes Dioxanyl,
gegebenenfalls substituiertes 1,3,4-Triazol-2-yl oder
gegebenenfalls substituiertes Tetrahydrofuryl, bedeutet.

23. Die Verwendung gemäss Anspruch 22 gegen die schädigende Wirkung herbizider Halogenacetanilide der Formel IV

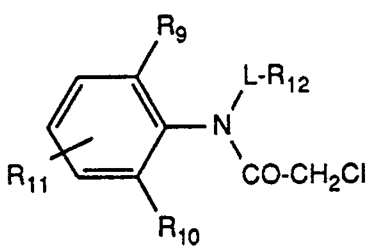

(IV)

worin
L eine $C_1$-$C_4$-Alkylenbrücke,
$R_9$, $R_{10}$ und $R_{11}$ unabhängig voneinander Wasserstoff, Halogen, $C_1$-$C_4$-Alkyl, $C_1$-$C_4$-Alkoxy, $C_1$-$C_4$-Halogenalkyl, $C_2$-$C_5$-Alkoxyalkyl oder $C_2$-$C_5$-Alkylthioalkyl, und
$R_{12}$ $C_1$-$C_4$-Alkoxy, -COOH, $C_1$-$C_4$-Alkoxycarbonyl, -$CONH_2$, Wasserstoff, $C_1$-$C_4$-Alkylcarbamoyl, Di-$C_1$-$C_4$-

alkylcarbamoyl, Cyan, $C_1$-$C_4$-Alkylcarbonyl, bedeutet.

24. Die Verwendung gemäss Anspruch 22 gegen die schädigende Wirkung von
N-Aethoxymethyl-N-chloracetyl-2-ethyl-6-methylanilin,
N-Chloracetyl-N-methoxymethyl-2,6-diethylanilin,
N-Chloracetyl-N-(2-methoxyethyl)-2,6-dimethylanilin,
N-Chloracetyl-N-(2-n-propoxyethyl)-2,6-dimethylanilin,
N-Chloracetyl-N-(2-isopropoxyethyl)-2,6-dimethylanilin,
N-Chloracetyl-N-(2-methoxyethyl)-2-ethyl-6-methylanilin,
N-Chloracetyl-N-(methoxyethyl)-2,6-diethylanilin,
N-(2-Ethoxyethyl)-N-chloracetyl-2-ethyl-6-methylanilin,
N-Chloracetyl-N-(2-methoxy-1-methylethyl)-2-methylanilin,
N-Chloracetyl-N-(2-methoxy-1-methylethyl)-2,6-dimethylanilin,
N-Chloracetyl-N-(2-methoxy-1-methylethyl)-2,6-diethylanilin,
N-Chloracetyl-N-(2-methoxy-1-methylethyl)-2-ethyl-6-methylani lin,
N-(2-Ethoxyethyl)-N-chloracetyl-2,6-diethylanilin,
N-Chloracetyl-N-(2-n-propoxyethyl)-2-ethyl-6-methylanilin,
N-Chloracetyl-N-(2-n-propoxyethyl)-2,6-diethylanilin,
N-Chloracetyl-N-(2-isopropoxyethyl)-2-ethyl-6-methylanilin,
N-Ethoxycarbonylmethyl-N-chloracetyl-2,6-dimethylanilin,
N-Ethoxycarbonylmethyl-N-chloracetyl-2,6-diethylanilin,
N-Chloracetyl-N-methoxycarbonylmethyl-2,6-dimethylanilin,
N-Chloracetyl-N-(2,2-diethoxyethyl)-2,6-dimethylanilin,
N-Chloracetyl-N-(2-methoxy-1-methylethyl)-2,3-dimethylanilin,
N-(2-Ethoxyethyl)-N-chloracetyl-2-methylanilin,
N-Chloracetyl-N-(2-methoxyethyl)-2-methylanilin,
N-Chloracetyl-N-(2-methoxy-2-methylethyl)-2,6-dimethylanilin,
N-(2-Ethoxy-2-methylethyl)-N-chloracetyl-2-ethyl-6-methylanilin,
N-Chloracetyl-N-(1-ethyl-1-methoxyethyl)-2,6-dimethylanilin,
N-Chloracetyl-N-(2-methoxyethyl)-2-methoxy-6-methylanilin,
N-n-Butoxymethyl-N-chloracetyl-2-tert.-butylanilin,
N-Chloracetyl-N-(2-ethoxyethyl-2-methylethyl)-2,6-dimethylanilin,
N-Chloracetyl-N-(-2-methoxyethyl)-2-chlor-6-methylanilin,
N-(2-Ethoxyethyl)-N-chloracetyl-2-chlor-6-methylanilin,
N-(2-Ethoxyethyl)-N-chloracetyl-2,3,6-trimethylanilin,
N-Chloracetyl-1-(2-methoxyethyl)-2,3,6-trimethylanilin,
N-Chloracetyl-N-cyanomethyl-2,6-dimethylanilin,
N-Chloracetyl-N-(1,3-dioxolan-2-ylmethyl)-2,6-dimethylanilin,
N-Chloracetyl-N-(1,3-dioxolan-2-ylmethyl)-2-ethyl-6-methylanilin,
N-Chloracetyl-N-(1,3-dioxan-2-ylmethyl)-2-ethyl-6-methylanilin,
N-Chloracetyl-N-(2-furylmethyl)-2,6-dimethylanilin,
N-Chloracetyl-N-(2-furylmethyl)-2-ethyl-6-methylanilin,
N-Chloracetyl-N-(2-tetrahydrofurylmethyl)-2,6-dimethylanilin,
N-Chloracetyl-N-(N,N-dimethylcarbamoylmethyl)-2,6-dimethylanilin,
N-(n-Butoxymethyl)-N-chloracetyl-2,6-diethylanilin,
N-(2-n-Butoxyethyl)-N-chloracetyl-2,6-diethylanilin,
N-Chloracetyl-N-(2-methoxy-1,2-dimethylethyl)-2,6-dimethylanilin,
N-Chloracetyl-N-isopropyl-2,3-dimethylanilin,
N-Chloracetyl-N-isopropyl-2-chloranilin,
N-Chloracetyl-N-(1H-pyrazol-1-ylmethyl)-2,6-dimethylanilin,
N-Chloracetyl-N-(1H-pyrazol-1-ylmethyl)-2-ethyl-6-methylanilin,
N-Chloracetyl-N-(1H-1,2,4-triazol-1-ylmethyl)-2,6-dimethylanilin,
N-Chloracetyl-N-(1H-1,2,4-triazol-1-ylmethyl)-2,6-diethylanilin,
N-Benzoylmethyl-N-chloracetyl-2,6-diethylanilin,
N-Benzoylmethyl-N-chloracetyl-2-ethyl-6-methylanilin,
N-Chloracetyl-N-(5-methyl-1,3,4-oxadiazol-2-yl)-2,6-diethylanilin,
N-Chloracetyl-N-(5-methyl-1,3,4-oxadiazol-2-yl)-2-ethyl-6-methylanilin,
N-Chloracetyl-N-(5-methyl-1,3,4-oxadiazol-2-yl)-2-tert.butylanilin,
N-Chloracetyl-N-(4-chlorbenzoylmethyl)-2,6-dimethylanilin und

N-Chloracetyl-N-(1-methyl-5-methylthio-1,3,4-triazol-2-ylmethyl)-2,6-diethylanilin.

25. Die Verwendung gemäss Anspruch 16 oder 17 gegen die schädigende Wirkung von N-Chloracetyl-N-(2-n-propoxyethyl)-2,6-diethylanilin.

26. Verfahren zum Schützen von Kulturpflanzen gegen die kulturpflanzenschädigende Wirkung von Herbiziden dadurch gekennzeichnet, dass man vor, während oder nach der Applikation des Herbizids die Kulturpflanze, deren Lebensraum, Teile der Kulturpflanze oder deren Samen oder Stecklinge der Kulturpflanze mit einer antagonistisch wirksamen Menge von 1-(3,5-Bis-trifluormethylphenyl)-2-thioxo-pyrrolidin-4-carbonsäurederivaten der Formel I

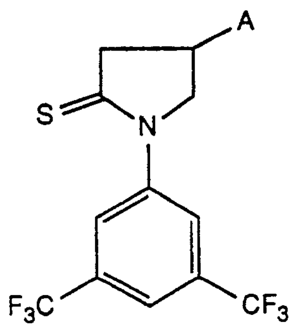

(I)

worin A die unter Anspruch 1 angegebene Bedeutung hat, behandelt.

27. Verfahren nach Anspruch 25, dadurch gekennzeichnet, dass A die unter Anspruch 2 angegebene Bedeutung hat.

28. Verfahren nach Anspruch 25 oder 26 zum Schützen von Kulturpflanzen gegen die schädigende Wirkung von Chloracetanilidherbiziden oder Sulfonylharnstoffherbiziden.

29. Verfahren nach Anspruch 25 zum Schützen von Reis, Sorghum und Mais.

30. Ein Pflanzenwuchs hemmendes Mittel, dadurch gekennzeichnet, dass es neben Träger-und/oder anderen Zuschlagstoffen als Wirkstoff mindestens ein Pyrrolidincarbonsäurederivat der Formel I, gemäss Anspruch 1, enthält.

31. Mittel gemäss Anspruch 30, dadurch gekennzeichnet, dass es zwischen 0,1 % und 95 % Wirkstoff der Formel I gemäss Anspruch 1 enthält.

32. Verfahren zur Hemmung des Pflanzenwachstums, dadurch gekennzeichnet, dass man einen Wirkstoff der Formel I, gemäss Anspruch 1, oder ein diesen Wirkstoff enthaltendes Mittel gemäss Anspruch 30 in einer wirksamen Menge auf die Pflanzen oder deren Lebensraum appliziert.

33. Verfahren gemäss Anspruch 32, dadurch gekennzeichnet, dass man eine Wirkstoffmenge zwischen 0,01 und 10 kg pro Hektar appliziert.

34. Verfahren zur Beeinflussung des Pflanzenwachstums zum Zweck der Ertragssteigerung, dadurch gekennzeichnet, dass man einen Wirkstoff der Formel I, gemäss Anspruch 1, oder ein diesen Wirkstoff enthaltendes Mittel gemäss Anspruch 30 in einer wirksamen Menge auf die Pflanzen oder deren Lebensraum appliziert.

Europäisches Patentamt

# EUROPÄISCHER RECHERCHENBERICHT

Nummer der Anmeldung

EP 90810339.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| D,A | EP - A2/A3 - 0 122 231 (CIBA-GEIGY AG) * Zusammenfassung * -- | 1,17, 21 | C 07 D 207/24<br>A 01 N 25/32 |
| D,A | EP - A2/A3 - 0 147 365 (CIBA GEIGY AG) * Zusammenfassung * -- | 1,17, 21 | |
| D,A | US - A - 4 013 445 (DANIEL BELLUS et al.) * Patentansprüche 1,7,8 * -- | 1,17 | |
| D,A | GB - A - 1 277 557 (GULF RESEARCH & DEVELOPMENT COMPANY) * Gesamt * ---- | 1,17, 19 | |

RECHERCHIERTE SACHGEBIETE (Int Cl 5)

C 07 D 207/00
A 01 N

Der vorliegende Recherchenbericht wurde für alle Patentansprüche erstellt

| Recherchenort | Abschlußdatum der Recherche | Prüfer |
|---|---|---|
| WIEN | 18-07-1990 | HEIN |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument